(19) Europäisches Patentamt
European Patent Office
Office européen des brevets

(11) **EP 4 410 314 A1**

(12) **EUROPEAN PATENT APPLICATION**

(43) Date of publication:
**07.08.2024 Bulletin 2024/32**

(21) Application number: **23154800.9**

(22) Date of filing: **02.02.2023**

(51) International Patent Classification (IPC):
***A61K 47/68*** *(2017.01)* ***A61K 31/4406*** *(2006.01)*
***A61K 45/06*** *(2006.01)* ***A61P 35/00*** *(2006.01)*

(52) Cooperative Patent Classification (CPC):
(C-Sets available)
**A61K 31/519; A61K 31/5517; A61K 45/06; A61K 47/68035; A61K 47/6849; A61K 47/6867; A61P 35/00** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **ADC Therapeutics SA**
  **1066 Epalinges (CH)**
- **MedImmune Limited**
  **Cambridge**
  **Cambridgeshire CB21 6GH (GB)**

(72) Inventor: **The designation of the inventor has not yet been filed**

(74) Representative: **Finnie, Nicholas James**
**ADC Therapeutics (UK) Limited**
**I-HUB, Imperial College White City Campus**
**84 Wood Lane**
**Shepherd's Bush**
**London W12 0BZ (GB)**

Remarks:
The complete document including Reference Table(s) and the Sequence Listing(s) can be downloaded from the EPO website

# (54) COMBINATION THERAPY COMPRISING ANTI-CD22 ANTIBODY-DRUG CONJUGATE AND IRAK1 INHIBITOR

(57) This disclosure relates to methods of treating proliferative disorders using a combination of (i) an antibody drug conjugate (ADC), wherein the drug is a pyrrolobenzodiazepine (PBD) dimer and the antibody is an anti-CD22 antibody; and (ii) an IRAK1 inhibitor, such as pacritinib.

Figure 1

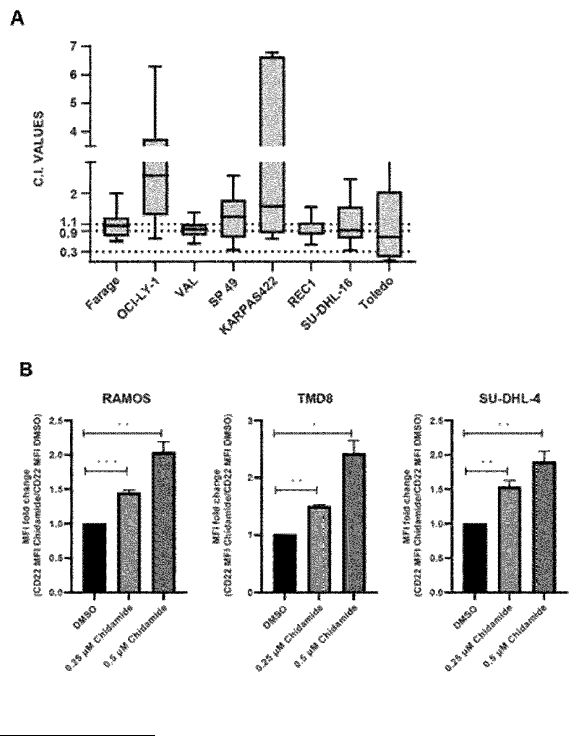


EP 4 410 314 A1

(52) Cooperative Patent Classification (CPC): (Cont.)

C-Sets
**A61K 31/519, A61K 2300/00;**
**A61K 31/5517, A61K 2300/00**

## Description

### Field

The present disclosure relates to novel dosage regimens for the treatment of pathological conditions, such as cancer, with Antibody Drug Conjugates (ADCs). In particular, the present disclosure relates to a combination of an anti-CD22, pyrrolobenzodiazepine dimer-based ADC with an IRAK1 inhibitor, such as pacritinib.

### Background

CD22 is a cell surface type I transmembrane sialoglycoprotein consisting of 7 extracellular immunoglobulin (Ig) domains and a 141-amino acid cytoplasmic tail. It is expressed on the surface of all stages of B-cell development, reaching peak levels on mature B cells. However, its expression is lost upon terminal differentiation to plasma cells. CD22 is not expressed on hematopoietic stem cells or other nonlymphoid hematopoietic or nonhematopoietic cells. This specific expression profile during B-cell development has made CD22 an attractive molecule for targeted B-cell malignancy therapies.

CD22 expression has been reported in follicular lymphoma, marginal zone lymphoma, mantle cell lymphoma (MCL), diffuse large B-cell lymphoma (DLBCL), small lymphocytic lymphoma, hairy cell leukemia, and chronic lymphocytic leukemia. Additionally, CD22 is almost universally expressed in B-cell acute lymphoblastic leukemia (B-ALL), including in the relapsed/refractory (R/R) setting. Various therapeutic modalities against CD22 have been developed, including naked or radiolabeled antibodies, bispecifics, and chimeric antigen receptor T-cell (CAR-T) therapy. However, outcomes in patients with relapsed or refractory B-cell acute lymphoblastic leukemia (R/R B-ALL) and lymphomas remain poor. In the CD22-targeting antibody drug conjugate field, while the development of the auristatin-based ADC pinatuzumab vedotin was discontinued for the treatment of non-Hodgkin lymphoma (NHL) after phase 2, the calicheamicin-based ADC inotuzumab ozogamicin was approved in 2017 for the treatment of adult patients with R/R B-ALL. However, its specific safety profile, which includes veno-occlusive disease (VOD), remains an ongoing area of concern.

Thus, there is an unmet need for novel therapies.

### Summary

CD22 is an attractive target for antibody-drug conjugates (ADCs) because it is highly expressed in lymphomas and leukemias such as R/R B-ALL and has rapid internalization kinetics. ADCT-602 is a novel ADC targeting CD22 that consists of humanized monoclonal antibody hLL2-C220, sitespecifically conjugated to the pyrrolobenzodiazepine (PBD) dimer-based payload tesirine. In preclinical studies, ADCT-602 demonstrated potent and specific cytotoxicity in CD22-expressing lymphoma and leukemia cell lines. ADCT-602 was specifically bound, internalized, and trafficked to lysosomes in CD22-expressing tumor cells and, following the release of the PBD dimer warhead, resulted in the formation of DNA interstrand crosslinks that persisted for 48 hours. In the presence of CD22-positive tumor cells, ADCT-602 caused bystander killing of CD22-negative tumor cells. *In vivo,* a single ADCT-602 dose resulted in potent, dose-dependent antitumor activity in subcutaneous and disseminated human lymphoma and leukemic models. Pharmacokinetic analyses (rat and cynomolgus monkey) showed excellent stability and tolerability of ADCT-602. In cynomolgus monkeys, B cells were efficiently depleted from circulation after one ADCT-602 dose. Gene signature association analysis revealed IRAK1 as a potential marker of resistance to ADCT-602. Combining ADCT-602 with the IRAK1 inhibitor, pacritinib, was beneficial in cell lines resistant to ADCT-602.

Accordingly the present disclosure is directed to methods of treating proliferative disorders, such as relapsed or refractory B-cell acute lymphoblastic leukemia, the methods comprising administration of a combination of (i) an ADC which targets CD22 and which has a PBD dimer as a cytotoxic agent and (ii) an IRAK1 inhibitor.

Accordingly, the present disclosure provides a method of treating a proliferative disorder in a subject which method comprises administering to the subject (i) an antibody drug conjugate (ADC), wherein the drug is a pyrrolobenzodiazepine (PBD) dimer and the antibody is an anti-CD22 antibody; and (ii) (a) an IRAK1 inhibitor or (b) a class I Histone Deacetylase (HDAC) inhibitor (HDI), which disorder is characterized by cells that express CD22.

In a related aspect the present disclosure provides (i) an antibody drug conjugate (ADC), wherein the drug is a pyrrolobenzodiazepine (PBD) dimer and the antibody is an anti-CD22 antibody; and (ii) (a) an IRAK1 inhibitor or (b) a class I Histone Deacetylase (HDAC) inhibitor (HDI) for use in the treatment of a proliferative disorder characterized by cells that express CD22.

In one embodiment, the PBD dimer is of formula I:

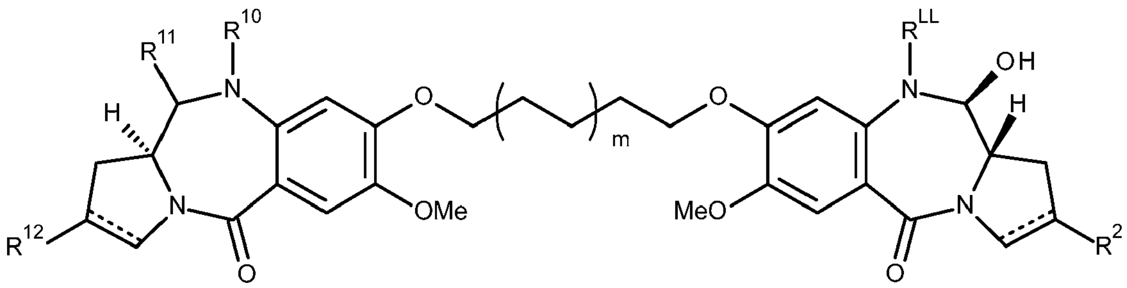

wherein:

(a) $R^{LL}$ is a linker for connection to Ab;
(b) (i) $R^{10}$ and $R^{11}$ together form a double bond between the nitrogen and carbon atoms to which they are attached; or (ii) $R^{10}$ is $R^{LLA}$ which is a linker for connection to Ab, and $R^{11}$ is OH, where $R^{LL}$ and $R^{LLA}$ may be the same or different; or (iii) $R^{10}$ is a capping group $R^C$ and $R^{11}$ is OH;
(c) m is 0 or 1; and
(d) when there is a double bond between C2 and C3, $R^2$ is methyl;

when there is a single bond between C2 and C3, $R^2$ is either H or

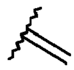

;

when there is a double bond between C2' and C3', $R^{12}$ is methyl;

when there is a single bond between C2' and C3', $R^{12}$ is H or

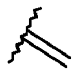

.

In one embodiment, the PBD dimer is of formula (III):

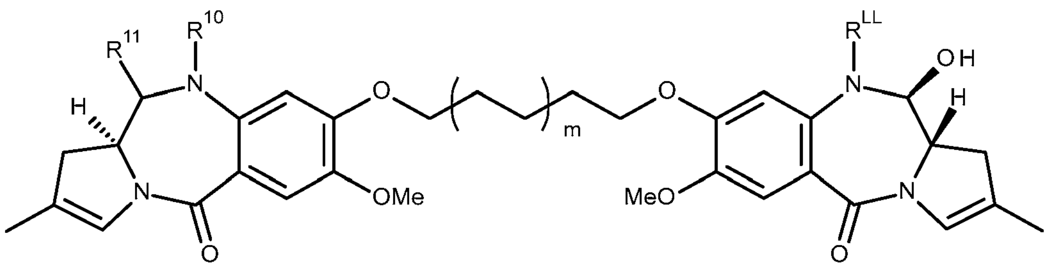

wherein:

(a) $R^{LL}$ is a linker for connection to Ab;
(b) (i) $R^{10}$ and $R^{11}$ together form a double bond between the nitrogen and carbon atoms to which they are attached; or (ii) $R^{10}$ is $R^{LLA}$ which is a linker for connection to Ab, and $R^{11}$ is OH; or (iii) $R^{10}$ is a capping group $R^C$, and $R^{11}$ is OH; and
(c) m is 0 or 1.

In one embodiment, the antibody comprises a VH domain having a CDR1 region with the sequence shown in SEQ ID NO.5; a CDR2 region with the sequence shown in SEQ ID NO.6; and a CDR3 region with the sequence shown

in SEQ ID NO.7; and a VL domain having a CDR1 region with the sequence shown in SEQ ID NO.8; a CDR2 region with the sequence shown in SEQ ID NO.9; and a CDR3 region with the sequence shown in SEQ ID NO.10. In a particular embodiment the antibody comprises a VH domain having the sequence according to SEQ ID NO. 1 and a VL domain having the sequence according to SEQ ID NO. 2.

In one embodiment the antibody comprises (i) heavy chains comprising an amino acid substitution of each of HC226 and HC229 according to EU numbering; (ii) light chains each having an amino acid substitution of the interchain cysteine residue κLC214 or λLC213 according to EU numbering; and (iii) heavy chains each retaining the unsubstituted interchain cysteine HC220 according to the EU numbering.

In one embodiment, the ADC is ADCx22 as defined below.

In one embodiment the IRAK1 inhibitor is pacritinib or a pharmaceutically acceptable salt, solvate, hydrate, cocrystal, or prodrug thereof.

In one embodiment the HDI is chidamide (Tucidinostat) or a pharmaceutically acceptable salt, solvate, hydrate, cocrystal, or prodrug thereof.

In one embodiment the proliferative disorder is a tumour which comprises cells that express CD22. In a particular embodiment the tumour comprises cells that overexpress IRAK1.

In one embodiment the tumour is resistant to monotherapy with an antibody drug conjugate (ADC) which comprises a pyrrolobenzodiazepine (PBD) dimer an anti-CD22 antibody.

The proliferative disorder may be selected from follicular lymphoma, marginal zone lymphoma, mantle cell lymphoma (MCL), diffuse large B-cell lymphoma (DLBCL), small lymphocytic lymphoma, hairy cell leukemia, and chronic lymphocytic leukemia.

Another aspect of the disclosure provides a kit comprising:

a first medicament comprising an anti-CD22 ADC;
a package insert comprising instructions for administration of the first medicament according to a method of treatment as disclosed herein. The kit may further comprise a second medicament comprising (a) an IRAK1 inhibitor or (b) a class I Histone Deacetylase (HDAC) inhibitor (HDI).

Another aspect of the disclosure provides a kit comprising:

a first medicament comprising an anti-CD22 ADC;
a second medicament comprising (a) an IRAK1 inhibitor or (b) an HDI; and, optionally,
a package insert comprising instructions for administration of the first medicament to an individual in combination with the second medicament for the treatment of a disorder.

Also provided by this aspect is a kit comprising a medicament comprising an anti-CD22 ADC and a package insert comprising instructions for administration of the medicament to an individual in combination with a composition comprising (a) an IRAK1 inhibitor or (b) an HDI for the treatment of a disorder.

Further provided by this aspect is a kit comprising a medicament comprising a PARP inhibitor and a package insert comprising instructions for administration of the medicament to an individual in combination with a composition comprising an anti-CD22 ADC for the treatment of a disorder.

## Detailed Description

### Anti-CD22/PBD Antibody Drug Conjugates

Anti-CD22/PBD Antibody Drug Conjugates for use in the methods and combinations described herein comprise an anti-CD22 binding agent, typically an anti-CD22 antibody, conjugated via a linker to a cytotoxic agent which is a pyrrolobenzodiazepine dimer.

### Antibodies

The term "antibody" herein is used in the broadest sense and specifically covers monoclonal antibodies, including both intact antibodies and antibody fragments, so long as they exhibit the desired biological activity, for example, the ability to bind a tumour antigen. Antibodies may be murine, rat, human, humanized, chimeric, or derived from other species. An antibody includes a full-length immunoglobulin molecule or an immunologically active portion of a full-length immunoglobulin molecule, *i.e.,* a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g. IgG, IgE, IgM, IgD,

and IgA), class (e.g. IgG1, IgG2, IgG3, IgG4, IgA1 and IgA2) or subclass, or allotype (e.g. human G1m1, G1m2, G1m3, non-G1m1 [that, is any allotype other than G1m1], G1m17, G2m23, G3m21, G3m28, G3m11, G3m5, G3m13, G3m14, G3m10, G3m15, G3m16, G3m6, G3m24, G3m26, G3m27, A2m1, A2m2, Km1, Km2 and Km3) of immunoglobulin molecule.

A variety of immunoglobulin variant formats are known in the art which are derived from conventional immunoglobulins, such as bispecific antibodies, scFvs, nanobodies and the like. These are all within the scope of the term "antibody" provided they retain the desired biological activity, for example, the ability to bind a tumour antigen.

*Anti-CD22 Antibodies*

The anti-CD22 antibodies used in the antibody drug conjugates bind to a CD22 polypeptide, typically a CD22 polypeptide which corresponds to Genbank accession no. BAB15489, version no. BAB15489.1 GI:10439338, record update date: Sep 11, 2006 11:24 PM. In one embodiment, the nucleic acid encoding CD22 polypeptide corresponds to Genbank accession no AK026467, version no. AK026467.1 GI:10439337, record update date: Sep 11, 2006 11:24 PM.

As used herein, "binds to a CD22 polypeptide" is used to mean the antibody binds CD22 with a higher affinity than a non-specific partner such as Bovine Serum Albumin (BSA, Genbank accession no. CAA76847, version no. CAA76847.1 GI:3336842, record update date: Jan 7, 201 1 02:30 PM). In some embodiments the antibody binds CD22 with an association constant ($K_a$) at least 2, 3, 4, 5, 10, 20, 50, 100, 200, 500, 1000, 2000, 5000, $10^4$, $10^5$ or $10^6$-fold higher than the antibody's association constant for BSA, when measured at physiological conditions. The antibodies of the invention can bind CD22 with a high affinity. For example, in some embodiments the antibody can bind CD22 with a $K_D$ equal to or less than about $10^{-6}$ M, such as $1 \times 10^{-6}$, $10^{-7}$, $10^{-8}$, $10^{-9}$, $10^{-10}$, $10^{-11}$, $10^{-12}$, $10^{-13}$ or $10^{-14}$.

In one embodiment the anti-CD22 antibody comprises a VH domain having a CDR1 region with the sequence shown in SEQ ID NO.5; a CDR2 region with the sequence shown in SEQ ID NO.6; and a CDR3 region with the sequence shown in SEQ ID NO.7; and a VL domain having a CDR1 region with the sequence shown in SEQ ID NO.8; a CDR2 region with the sequence shown in SEQ ID NO.9; and a CDR3 region with the sequence shown in SEQ ID NO.10.

Preferably the antibody comprises a VH domain having the sequence according to SEQ ID NO. 1. Preferably the antibody comprises a VL domain having the sequence according to SEQ ID NO. 2. Most preferably the antibody comprises a heavy chain having the sequence according to SEQ ID NO. 3 and a light chain having the sequence according to SEQ ID NO. 4, optionally wherein the drug moiety is conjugated to the cysteine at position 219 of SEQ ID NO.3.

"Antibody fragments" comprise a portion of a full length antibody, generally the antigen binding or variable region thereof. Examples of antibody fragments include Fab, Fab', $F(ab')_2$, and scFv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method or may be made by recombinant DNA methods. The monoclonal antibodies may also be isolated from phage antibody libraries or from transgenic mice carrying a fully human immunoglobulin system.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity. Chimeric antibodies include "primatized" antibodies comprising variable domain antigen-binding sequences derived from a non-human primate (e.g. Old World Monkey or Ape) and human constant region sequences.

An "intact antibody" herein is one comprising VL and VH domains, as well as a light chain constant domain (CL) and heavy chain constant domains, CH1, CH2 and CH3. The constant domains may be native sequence constant domains (e.g. human native sequence constant domains) or amino acid sequence variant thereof. The intact antibody may have one or more "effector functions" which refer to those biological activities attributable to the Fc region (a native

sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; and down regulation of cell surface receptors such as B cell receptor and BCR.

*Modification of antibodies*

The antibodies disclosed herein may be modified. For example, to make them less immunogenic to a human subject. This may be achieved using any of a number of techniques familiar to the person skilled in the art. Such techniques includes humanisation to reduce the *in vivo* immunogenicity of a non-human antibody or antibody fragment. There are a range of humanisation techniques, including 'CDR grafting', 'guided selection', 'deimmunization', 'resurfacing' (also known as 'veneering'), 'composite antibodies', 'Human String Content Optimisation' and framework shuffling.

The antibody may be a fully human monoclonal IgG1 antibody, preferably IgG1,κ. In one embodiment is has the allotype G1m17/G1m(z).

In a particular embodiment, the antibody has been engineered as described in WO206/166307 to modify the interchain cysteines, to reduce the number the cysteine residues available for conjugation. In a specific embodiment, the heavy chain comprises an amino acid substitution of each of HC226 and HC229 according to the EU numbering (corresponding to position 219 of SEQ ID NO.3). The substitution may be a C->V substitution. The heavy chain retains an unsubstituted interchain cysteine HC220 (corresponding to position 219 of SEQ ID NO.3). In one embodiment the light chain also comprises an amino acid substitution of each of LC214 according to the EU numbering (corresponding to position 219 of SEQ ID NO.4). The substitution may be a C->S substitution.

PBD Cytotoxin

Pyrrolobenzodiazepines (PBDs) suitable for use in the present disclosure in some embodiments have the ability to recognise and bond to specific sequences of DNA; the preferred sequence is PuGPu. PBDs are of the general structure:

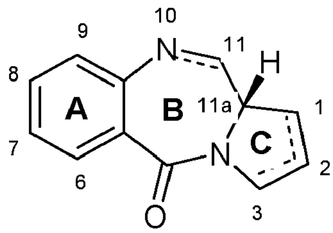

They differ in the number, type and position of substituents, in both their aromatic A rings and pyrrolo C rings, and in the degree of saturation of the C ring. In the B-ring there is either an imine (N=C), a carbinolamine(NH-CH(OH)), or a carbinolamine methyl ether (NH-CH(OMe)) at the N10-C11 position which is the electrophilic centre responsible for alkylating DNA. All of the known natural products have an (S)-configuration at the chiral C11a position which provides them with a right-handed twist when viewed from the C ring towards the A ring. This gives them the appropriate three-dimensional shape for isohelicity with the minor groove of B-form DNA, leading to a snug fit at the binding site). Their ability to form an adduct in the minor groove, enables them to interfere with DNA processing, hence their use as antitumour agents.

The cytotoxin is typically a PBD dimer, such as a PBD dimer, which together with the linker(s) and any optional capping group, has the following formula (I):

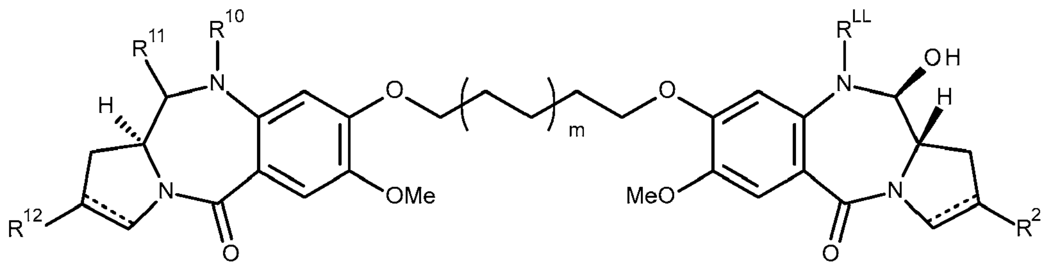

wherein $R^{LL}$ is a linker for connection to the antibody, with examples and particular embodiments provided in more detail below in the section entitled 'Linkers'.

When released from the linker $R^{LL}$, the bond between N10, to which $R^{LL}$ was originally attached, and C11 typically together form a double bond between the nitrogen and carbon atoms to which they are attached (an imine (C=N)). Similarly, in the released drug, $R^{10}$ and $R^{11}$ typically together form a double bond between the nitrogen and carbon atoms to which they are attached. Thus the released drug may be of formula RelA:

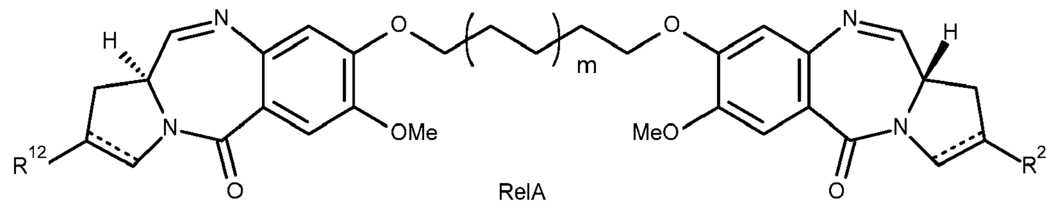

RelA

*R10 and R11*

In some embodiments, $R^{10}$ and $R^{11}$ together form a double bond between the nitrogen and carbon atoms to which they are attached.

In another embodiment, $R^{10}$ is a linker $R^{LLA}$ for connection to the antibody, Ab, and $R^{11}$ is OH, with $R^{LLA}$ having the same definition as $R^{LL}$, and $R^{LLA}$ and $R^{LL}$ being the same or different.

In another embodiment, $R^{10}$ is a capping group $R^C$ and $R^{11}$ is OH.

A capping group can be used to reduce or inhibit the cytotoxic activity of the PBD, effectively forming a prodrug, but in this context is not linked to the cell binding agent. The capping group is then removed, for example in the target cell or in the tumour microenviroment, to activate the drug. Various capping groups are described in Franzyk and Christiansen, 2021, Molecules 26: 1292. (1) Prodrugs cleaved in acidic media e.g. salts of dithiocarbamates. (2) Prodrugs cleaved by reactive oxygen species. (3) Prodrugs cleaved by glutathione. (4) Prodrugs cleaved by expressed enzymes, such as oxidoreductases, hydrolases and matrix metalloproteinases. (5) Prodrugs cleaved by betaglucuronidase, e.g. $R^C$ may comprise a glucuronide, for example:

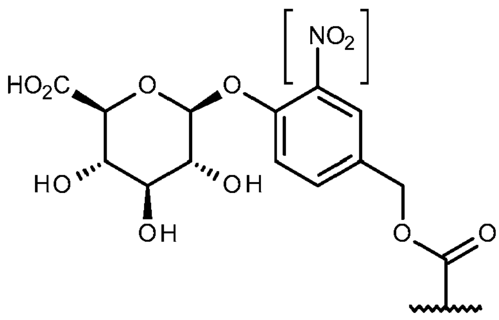

Wherein the square brackets indicate the $NO_2$ group is optional. In one embodiment, the $NO_2$ group is present.

A capping group may also be used to modify the physicochemical characteristics of the antibody drug conjugate e.g. to make it more stable. For example, the capping group may increase the hydrophilicity of the antibody drug conjugate.

*m*

In some embodiments, m is 0. In some embodiments, m is 1.

*R2 and R12*

In some embodiments, $R^2$ and $R^{12}$ are the same.

In some embodiments, there is a double bond between C2 and C3 and between C2' and C3, and $R^2$ and $R^{12}$ are both methyl.

In some embodiments, there is a single bond between C2 and C3 and between C2' and C3, and $R^2$ and $R^{12}$ are both H.

In some embodiments, there is a single bond between C2 and C3 and between C2' and C3, and $R^2$ and $R^{12}$ are both

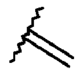

.

In one embodiment, the drug linker, L-D is of formula (II)

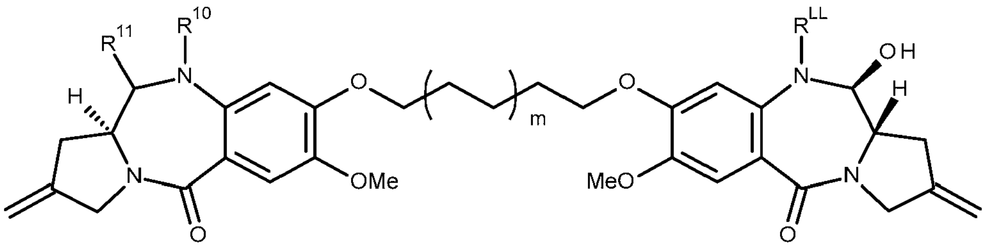

wherein $R^{LL}$, $R^{10}$, $R^{11}$ and m are as defined above.

In a particular embodiment, $R^{10}$, and $R^{11}$ together form a double bond between the nitrogen and carbon atoms to which they are attached; and m is 0 (i.e. SG2000 with a linker at N10).

In another embodiment, the drug-linker, L-D is of formula (III):

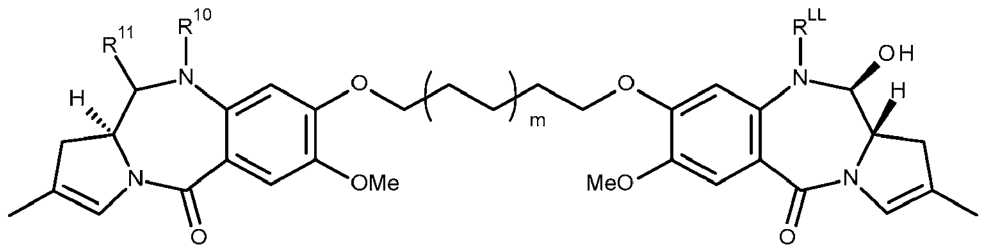

where $R^{LL}$, $R^{10}$, $R^{11}$ and m are as defined above.

In a particular embodiment, $R^{10}$, and $R^{11}$ together form a double bond between the nitrogen and carbon atoms to which they are attached; and m is 1 (i.e. SG3199 with a linker at N10).

<u>Drug-linkers</u>

A wide variety of linker technologies are available in the art to link cytotoxins to cell binding agents. Linkers can incorporate various different moieties to assist with antibody-drug conjugate stability and determine drug release characteristics. For example the linker may include a cleavable moiety, such as one that is cleavable by cathepsin B (e.g. Valine-Alanine or Valine-Citrulline). Another strategy is to use a pH-sensitive linker whereby the lower pH of the endosome and lysosome compartments the hydrolysis of an acid-labile group within the linker, such as a hydrazone. Alternative a linker may be non-cleavable, which can avoid or reduce off-target effects and improve plasma stability during circulation.

The functionality that allows conjugation to the cell binding agent is based on the site of conjugation and its chemistry. N-hydroxysuccinimide esters are a common choice for functionalizing amines, especially when coupling to ε-lysine residues. For conjugation to cysteines, thiol-reactive maleimide is the most applied reactive handle, although it is also possible to create a disulfide bridge by oxidation with a linker bearing a sulfhydryl group. Aldehyde or keto functional groups such as oxidized sugar groups or pAcPhe unnatural amino acids can be reacted with hydrazides and alkoxyamines to yield acid-labile hydrazones or oxime bonds. In addition, a hydrazine can be coupled with an aldehyde via HIPS ligation to generate a stable C-C linkage.

More recent approaches have been based on the N-linked glycosylation site in antibodies, such as Asn-297 in IgG molecules. GlycoConnect™ (Synaffix), using enzymes to trim the N-linked glycans to a GlcNAc core and then a further enzymatic process to introduce an activated moiety comprising azide which can then be used to incorporate the drug-linker using copper-free click chemistry.

Other aspects of linker chemistry include spacers and/or moieties which mask the hydrophobicity of the cytotoxin payload, reduce cellular efflux mechanisms and/or increase overall stability, such as a polyethylene glycol (PEG) chain within the linker or a polar functional group such as a sulphonyl.

In some embodiments, the antibody drug conjugates of the disclosure can be described as Ab-L-D, e.g. where Ab is an anti-CD22 antibody, D is the PBD-containing cytotoxin and L is a linker. The number of Drug moieties per Ab

(the drug loading, p) depends on the number of linkers attached to each Ab, and the number of Drug moieties per linker. Typically the drug loading, p, is from 1 to 8, such as from 1 to 4, 2 to 3, 2 to 4 or 1 to 2. In some embodiments one Drug moiety is joined to each linker whereas in others, more than one Drug moiety may be joined to each linker (e.g. a branched linker). Drug loading is typically considered on an average basis since variations can arise from the conjugation process. Methods for determining average drug loading are known in the art.

In one embodiment the linker (e.g. shown as $R^{LL}$ in formulas (I), (II) and (III)) is of formula (Ia):

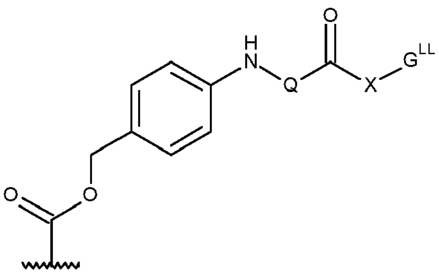

,

wherein

Q is:

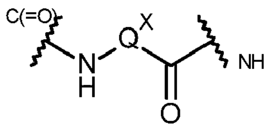

,

where $Q^X$ is such that Q is an amino-acid residue, a dipeptide residue, a tripeptide residue or a tetrapeptide residue;

X is:

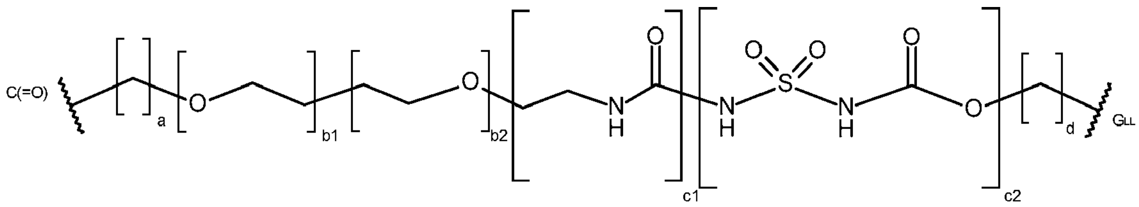

where a = 0 to 5, b1 = 0 to 16, b2 = 0 to 16, c1 = 0 or 1, c2 = 0 or 1, d = 0 to 5, wherein at least b1 or b2 = 0 (i.e. only one of b1 and b2 may not be 0) and at least c1 or c2 = 0 (i.e. only one of c1 and c2 may not be 0); and

$G^{LL}$ is a linker group connected to Ab;

*$Q^X$*

In one embodiment, Q is an amino acid residue. The amino acid may be a natural amino acid or a non-natural amino acid.

In one embodiment, Q is selected from: Phe, Lys, Val, Ala, Cit, Leu, Ile, Arg, and Trp, where Cit is citrulline.

In one embodiment, Q comprises a dipeptide residue. The amino acids in the dipeptide may be any combination of natural amino acids and non-natural amino acids. In some embodiments, the dipeptide comprises natural amino acids. Where the linker is a cathepsin labile linker, the dipeptide is the site of action for cathepsin-mediated cleavage. The dipeptide then is a recognition site for cathepsin.

In one embodiment, Q is selected from:

$^{NH}$-Phe-Lys-$^{C=O}$, $^{NH}$-Val-Ala-$^{C=O}$, $^{NH}$-Val-Lys-$^{C=O}$, $^{NH}$-Ala-Lys-$^{C=O}$, $^{NH}$-Val-Cit-$^{C=O}$, $^{NH}$-Phe-Cit-$^{C=O}$, $^{NH}$-Leu-Cit-$^{C=O}$, $^{NH}$-Ile-Cit-$^{C=O}$, $^{NH}$-Phe-Arg-$^{C=O}$, $^{NH}$-Trp-Cit-$^{C=O}$, and

$^{NH}$-Gly-Val-$^{C=O}$;

where Cit is citrulline.

Preferably, Q is selected from:

$^{NH}$-Phe-Lys-$^{C=O}$, $^{NH}$-Val-Ala-$^{C=O}$, $^{NH}$-Val-Lys-$^{C=O}$, $^{NH}$-Ala-Lys-$^{C=O}$, and $^{NH}$-Val-Cit-$^{C=O}$.

Most preferably, Q is selected from $^{NH}$-Phe-Lys-$^{C=O}$, $^{NH}$-Val-Cit-$^{C=O}$ or $^{NH}$-Val-Ala-$^{C=O}$.

Other dipeptide combinations of interest include:

$^{NH}$-Gly-Gly-$^{C=O}$, $^{NH}$-Gly-Val-$^{C=O}$, $^{NH}$-Pro-Pro-$^{C=O}$, and $^{NH}$ -Val-Glu- $^{C=O}$.

Other dipeptide combinations may be used, including those described by Dubowchik et al., Bioconjugate Chemistry, 2002, 13,855-869, which is incorporated herein by reference.

In some embodiments, Q is a tripeptide residue. The amino acids in the tripeptide may be any combination of natural amino acids and non-natural amino acids. In some embodiments, the tripeptide comprises natural amino acids. Where the linker is a cathepsin labile linker, the tripeptide is the site of action for cathepsin-mediated cleavage. The tripeptide then is a recognition site for cathepsin.

Tripeptide linkers of particular interest are:

$^{NH}$-Glu-Val-Ala-$^{C=O}$, $^{NH}$-Glu-Val-Cit-$^{C=O}$, $^{NH}$-$\alpha$Glu-Val-Ala-$^{C=O}$ and $^{NH}$-$\alpha$Glu-Val-Cit-$^{C=O}$

Tetrapeptide linkers may also be used, such as those based on glycine and phenylalanine.

In the above representations of peptide residues, NH- represents the N-terminus, and -C=O represents the C-terminus of the residue. The C-terminus binds to the NH attached to the benzene ring.

Glu represents the residue of glutamic acid, i.e.:

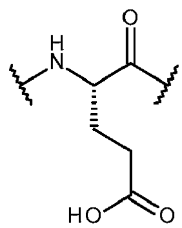

$\alpha$Glu represents the residue of glutamic acid when bound via the $\alpha$-chain, i.e.:

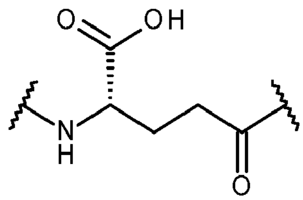

.

In one embodiment, the amino acid side chain is chemically protected, where appropriate. The side chain protecting group may be a group as discussed above. Protected amino acid sequences are cleavable by enzymes. For example, a dipeptide sequence comprising a Boc side chain-protected Lys residue is cleavable by cathepsin.

Protecting groups for the side chains of amino acids are well known in the art and are described in the Novabiochem Catalog, and as described above.

*$G^{LL}$*

$G^{LL}$ may be selected from:

| | | | |
|---|---|---|---|
| ($G^{LL1-1}$) | 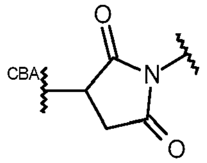 | ($G^{LL8-1}$) | 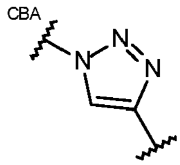 |
| ($G^{LL1-2}$) | 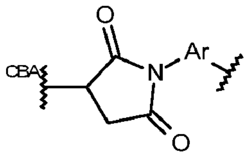 | ($G^{LL8-2}$) | 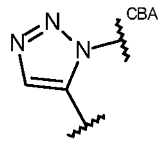 |
| ($G^{LL2}$) | 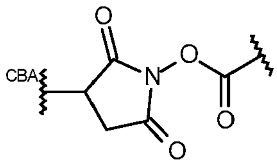 | ($G^{LL9-1}$) | 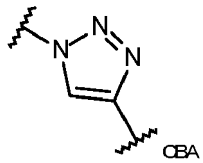 |
| ($G^{LL3-1}$) | 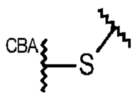 | ($G^{LL9-2}$) | 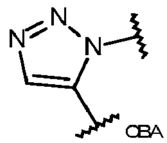 |
| ($G^{LL3-2}$) | 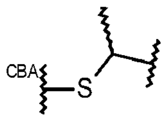 | ($G^{LL10}$) | 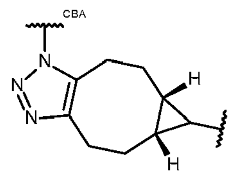 |
| ($G^{LL-4}$) | 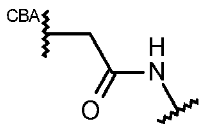 | ($G^{LL11}$) | 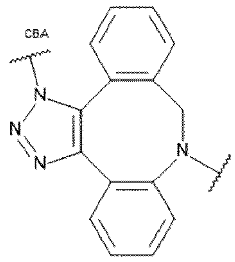 |
| ($G^{LL5}$) | 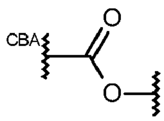 | ($G^{LL12}$) | 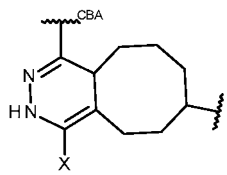 |
| ($G^{LL6}$) | 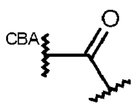 | ($G^{LL13}$) | 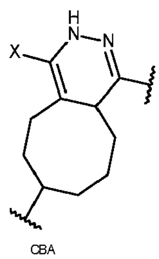 |

(continued)

| | | | |
|---|---|---|---|
| ($G^{LL7}$) | 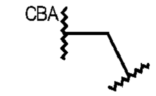 | ($G^{LL14}$) | 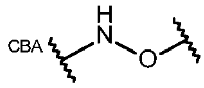 |

where Ar represents a $C_{5-6}$ arylene group, e.g. phenylene and X represents $C_{1-4}$ alkyl. CBA indicates the end of $G^{LL}$ connected to the antibody.

In some embodiments, $G^{LL}$ is selected from $G^{LL1-1}$ and $G^{LL1-2}$. In some of these embodiments, $G^{LL}$ is GLL1-1.

In other embodiments, $G^{LL}$ is selected from $G^{LL10}$ and $G^{LL11}$. In some of these embodiments, $G^{LL}$ is $G^{LL10}$.

$C_{5-6}$ arylene: The term "$C_{5-6}$ arylene", as used herein, pertains to a divalent moiety obtained by removing two hydrogen atoms from an aromatic ring atom of an aromatic compound.

In this context, the prefixes (e.g. $C_{5-6}$) denote the number of ring atoms, or range of number of ring atoms, whether carbon atoms or heteroatoms.

The ring atoms may be all carbon atoms, as in "carboarylene groups", in which case the group is phenylene ($C_6$).

Alternatively, the ring atoms may include one or more heteroatoms, as in "heteroarylene groups".

Examples of heteroarylene groups include, but are not limited to, those derived from:

$N_1$: pyrrole (azole) ($C_5$), pyridine (azine) ($C_6$);
$O_1$: furan (oxole) (Cs);
$S_1$: thiophene (thiole) (Cs);
$N_1O_1$: oxazole (Cs), isoxazole (Cs), isoxazine ($C_6$);
$N_2O_1$: oxadiazole (furazan) ($C_5$);
$N_3O_1$: oxatriazole (Cs);
$N_1S_1$: thiazole (Cs), isothiazole (Cs);
$N_2$: imidazole (1,3-diazole) (Cs), pyrazole (1,2-diazole) (Cs), pyridazine (1,2-diazine) ($C_6$), pyrimidine (1,3-diazine) ($C_6$) (e.g., cytosine, thymine, uracil), pyrazine (1,4-diazine) ($C_6$); and $N_3$: triazole (Cs), triazine ($C_6$).

$C_{1-4}$ alkyl: The term "$C_{1-4}$ alkyl" as used herein, pertains to a monovalent moiety obtained by removing a hydrogen atom from a carbon atom of a hydrocarbon compound having from 1 to 4 carbon atoms, which may be aliphatic or alicyclic, and which may be saturated or unsaturated (e.g. partially unsaturated, fully unsaturated). The term "$C_{1-n}$ alkyl" as used herein, pertains to a monovalent moiety obtained by removing a hydrogen atom from a carbon atom of a hydrocarbon compound having from 1 to n carbon atoms, which may be aliphatic or alicyclic, and which may be saturated or unsaturated (e.g. partially unsaturated, fully unsaturated). Thus, the term "alkyl" includes the sub-classes alkenyl, alkynyl, cycloalkyl, etc., discussed below.

*X*

X is:

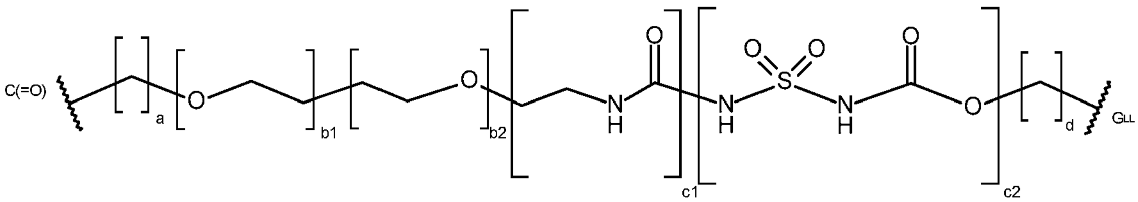

where a = 0 to 5, b1 = 0 to 16, b2 = 0 to 16, c1 = 0 or 1, c2 = 0 or 1, d = 0 to 5, wherein at least b1 or b2 = 0 and at least c1 or c2 = 0.

a may be 0, 1, 2, 3, 4 or 5. In some embodiments, a is 0 to 3. In some of these embodiments, a is 0 or 1. In further embodiments, a is 0. In further embodiments, a is 1.

b1 may be 0, 1, 2, 3, 4, 5, 6, 7, 8, 9, 10, 11, 12, 13, 14, 15 or 16. In some embodiments, b1 is 0 to 12. In some of these embodiments, b1 is 0 to 8, and may be 0, 2, 3, 4, 5 or 8. In further embodiments, b1 is 2.

b2 may be 0, 1, 2, 3, 4, 5, 6, 7, 8, 9, 10, 11, 12, 13, 14, 15 or 16. In some embodiments, b2 is 0 to 12. In some of these embodiments, b2 is 0 to 8, and may be 0, 2, 3, 4, 5 or 8. In further embodiments, b2 is 8.

Only one of b1 and b2 may not be 0.

c1 may be 0 or 1, preferably 1.

c2 may be 0 or 1, preferably 0.

Only one of c1 and c2 may not be 0.

d may be 0, 1, 2, 3, 4 or 5. In some embodiments, d is 0 to 3. In some of these embodiments, d is 1 or 2. In further embodiments, d is 2. In further embodiments, d is 5.

In some embodiments of X, a is 0, b1 is 0, c1 is 1, c2 is 0 and d is 2, and b2 may be from 0 to 8. In some of these embodiments, b2 is 0, 2, 3, 4, 5 or 8. In further embodiments, b2 is 8.

In some embodiments of X, a is 1, b2 is 0, c1 is 0, c2 is 1, d is 2, and b1 may be from 0 to 8. In some of these embodiments, b1 is 0, 2, 3, 4, 5 or 8. In further embodiments, b1 is 2.

The linker is typically connect to the PBD dimer via the N10 position, such as is shown in the location of $R^{LL}$ in the example embodiments below.

Drug Linker embodiments

In some embodiments, the drug-linker, L-D, is selected from:

| | |
|---|---|
| A1 | 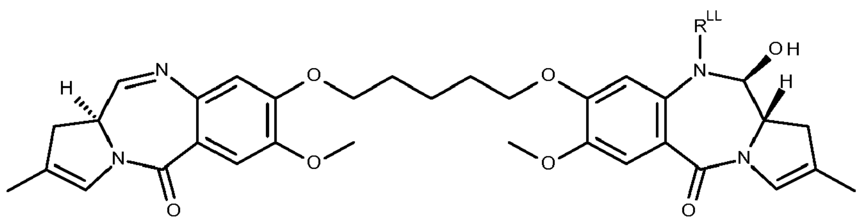 |
| A2 | 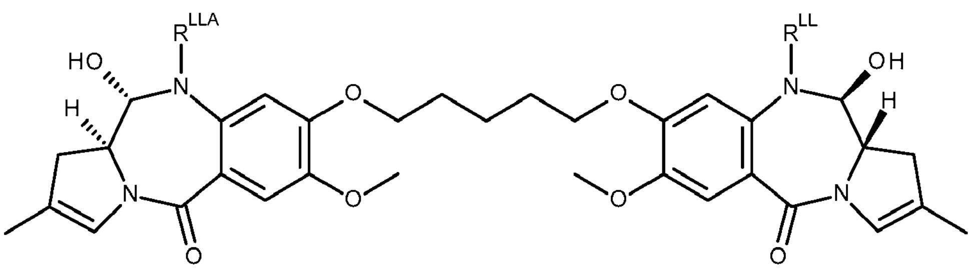 |

where $R^{LL}$ and $R^{LLA}$ are as described above.

In some embodiments, L-D is:

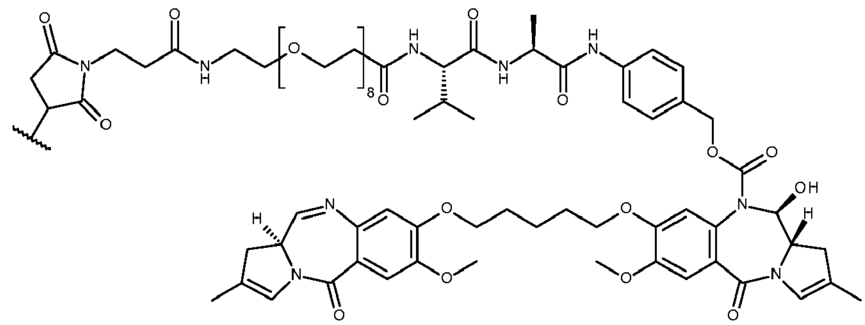

Site of Conjugation and Drug Loading

Drug-linkers can be conjugated to a cell binding agent, such as an antibody, using a variety of methods known in the art and at a number of different sites. Conjugation sites include cysteine residues and lysine residues in the antibody sequence (endogenous or engineered), as well as sites of N-linked glycosylation following trimming (e.g. the GlycoConnect™ or GlyClick™ approaches). Thus in one embodiment the drug-linker is conjugated via a trimmed Asn-GlcNAc residue, typically at the endogenous N-linked glycosylation site in the antibody (Asn-297). With respect to cysteine conjugation, in one embodiment the cysteine is an endogenous cysteine located in the hinge region or Fc

domain. In another embodiment the cysteine in an engineered cysteine introduced in the hinge region or Fc domain.

The drug loading is the average number of PBD drugs per cell binding agent, e.g. antibody.

The average number of drugs per antibody in preparations of ADC from conjugation reactions may be characterized by conventional means such as UV, reverse phase HPLC, HIC, mass spectroscopy, ELISA assay, and electrophoresis. The quantitative distribution of ADC in terms of p may also be determined. By ELISA, the averaged value of p in a particular preparation of ADC may be determined (Hamblett et. al. (2004) Clin. Cancer Res. 10:7063-7070; Sanderson et. al. (2005) Clin. Cancer Res. 11:843-852). However, the distribution of p (drug) values is not discernible by the antibody-antigen binding and detection limitation of ELISA. Also, ELISA assay for detection of antibody-drug conjugates does not determine where the drug moieties are attached to the antibody, such as the heavy chain or light chain fragments, or the particular amino acid residues. In some instances, separation, purification, and characterization of homogeneous ADC where p is a certain value from ADC with other drug loadings may be achieved by means such as reverse phase HPLC or electrophoresis. Such techniques are also applicable to other types of conjugates.

For some antibody-drug conjugates, p may be limited by the number of attachment sites on the antibody. For example, an antibody may have only one or several cysteine thiol groups, or may have only one or several sufficiently reactive thiol groups through which a linker may be attached. Higher drug loading, e.g. p >5, may cause aggregation, insolubility, toxicity, or loss of cellular permeability of certain antibody-drug conjugates.

Typically, fewer than the theoretical maximum of drug moieties are conjugated to an antibody during a conjugation reaction. An antibody may contain, for example, many lysine residues that do not react with the drug-linker intermediate or linker reagent. Only the most reactive lysine groups may react with an amine-reactive linker reagent. Also, only the most reactive cysteine thiol groups may react with a thiol-reactive linker reagent. Generally, antibodies do not contain many, if any, free and reactive cysteine thiol groups which may be linked to a drug moiety. Most cysteine thiol residues in the antibodies of the compounds exist as disulfide bridges and must be reduced with a reducing agent such as dithiothreitol (DTT) or TCEP, under partial or total reducing conditions. The loading (drug/antibody ratio) of an ADC may be controlled in several different manners, including: (i) limiting the molar excess of drug-linker intermediate or linker reagent relative to antibody, (ii) limiting the conjugation reaction time or temperature, and (iii) partial or limiting reductive conditions for cysteine thiol modification.

Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by engineering one, two, three, four, or more cysteine residues (e.g., preparing mutant antibodies comprising one or more non-native cysteine amino acid residues). US 7,521,541 teaches engineering antibodies by introduction of reactive cysteine amino acids.

Cysteine amino acids may be engineered at reactive sites in an antibody, and which do not form intrachain or intermolecular disulfide linkages (US 7,521,541; US 7,723,485; WO2009/052249). The engineered cysteine thiols may react with linker reagents or the drug-linker reagents of the present disclosure which have thiol-reactive, electrophilic groups such as maleimide or alpha-halo amides to form ADC with cysteine engineered antibodies and the PBD drug moieties. The location of the drug moiety can thus be designed, controlled, and known. The drug loading can be controlled since the engineered cysteine thiol groups typically react with thiol-reactive linker reagents or drug-linker reagents in high yield. Engineering an IgG antibody to introduce a cysteine amino acid by substitution at a single site on the heavy or light chain gives two new cysteines on the symmetrical antibody. Cysteines may also be removed by mutation, for example to limit the number of sites available for conjugation where a cysteine-based approach is used, and therefore control the drug to antibody ratio by limiting the number of different species that can be formed.

Where more than one nucleophilic or electrophilic group of the antibody reacts with a drug-linker intermediate, or linker reagent followed by drug moiety reagent, then the resulting product is a mixture of ADC compounds with a distribution of drug moieties attached to an antibody, e.g. 1, 2, 3, etc. Liquid chromatography methods such as polymeric reverse phase (PLRP) and hydrophobic interaction (HIC) may separate compounds in the mixture by drug loading value. Preparations of ADC with a single drug loading value (p) may be isolated, however, these single loading value ADCs may still be heterogeneous mixtures because the drug moieties may be attached, via the linker, at different sites on the antibody.

Thus the antibody-drug conjugate compositions of the disclosure include mixtures of antibody-drug conjugate compounds where the antibody has one or more PBD drug moieties and where the drug moieties may be attached to the antibody at various amino acid residues.

In one embodiment, the average number of dimer pyrrolobenzodiazepine groups per antibody is in the range 1 to 8. In some embodiments the range is selected from 1 to 4, 2 to 4, 1 to 3 and 1 to 2. In a particular embodiment where cysteines have been engineered as described above to reduce the available cysteines for conjugation to one on each chain, the average number of dimer pyrrolobenzodiazepine groups per antibody is in the range 1.4 to 2, such as 1.4 or 1.5, 1.6 or 1.7 to 1.9 (each antibody drug conjugate molecule will have either one or two PBDs conjugated).

In some embodiments, there are one or two dimer pyrrolobenzodiazepine groups per antibody.
Where L-D has two linking groups, these are preferably to the same antibody. In some of these embodiments, only one L-D is attached to each antibody, so the drug loading is 1.

Preparation of Drug conjugates

The antibody drug conjugates of the present disclosure may be prepared by conjugating the drug-linker, such as the following drug linker (of formula (I) - as previously defined herein) to the antibody:

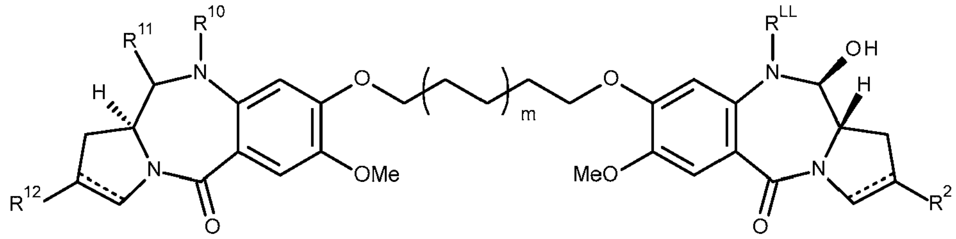

As described above, a number of conjugation techniques are known in the art, such as (i) conjugation to an endogenous or engineered cysteine residue via maleimide, as for example described in US9,889,207 or Flynn et al., 2016. Mol Cancer Ther 15: 2709 - as would be applicable to compound C2 below; and (ii) using GlyClick or GlycoConnect to attached via chemoenzymatically-trimmed N-linked glycosylation site, e.g. at Asn-297 or its equivalent, as described in WO2018/146188 which describes the use of EndoS to trim glycan isoforms to core GlcNAc, followed by enzymatic transfer to the core GlcNAc of a N-acetylgalactose (GalNAc) residue harboring an azide group for conjugation to the drug linker, typically using Galactose Transferase (GalT) or Galactose-N-acetyl Transferase (GalNAcT) enzyme. If a GalT enzyme is used, preferably the enzyme incorporates the Y289L and/or the C342T. Finally, the drug-linker is reacted with the azide group using copper-free click chemistry, such as the method described in van Geel, R., et al., Bioconjugate Chemistry, 2015, 26, 2233-2242. This method would be applicable to compound C1 below.
The drug linker may be synthesised as described in, for example, Tibergien et al., 2016, ACS Med. Chem. Lett. 7: 983-987, WO2014/057074, WO2018/069490 and WO2018/146188.
A drug-linker of particular interest is described in WO2014/057074 (compound 24).

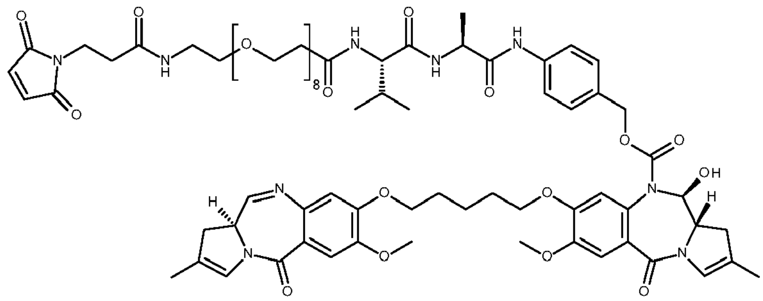

Preferred PBD agents

ADCx22

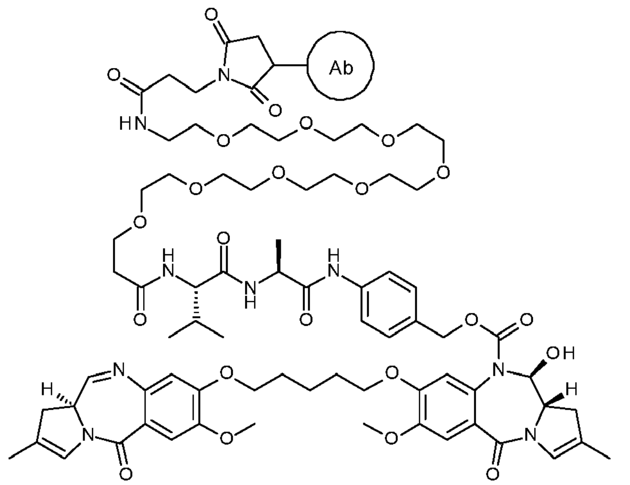

wherein "Ab" represents" represents antibody EMabC220. This antibody comprises a heavy chain having the sequence according to SEQ ID NO. 3 and a light chain having the sequence according to SEQ ID NO. 4. Linkage to the drug occurs on Heavy Chain interchain cysteine Cys220 (EU numbering). HC220 corresponds to position 219 of SEQ ID NO.3.

It is noted that "having the sequence" has the same meaning as "comprising the sequence"; in particular, in some embodiments the heavy chain of ADCx22 is expressed with an additional terminal 'K' residue (so, ending ...SPG**K**), with the terminal K being optionally removed post-translationally to improve the homogeneity of the final therapeutic ADC product.

IRAK1 Inhibitors

Interleukin-1 receptor-associated kinase 1 (IRAK1 - UniProt P51617) is a serine-threonine protein kinase plays an important role in the regulation of the expression of inflammatory genes by immune cells, such as monocytes and macrophages, which in turn help the immune system in eliminating bacteria, viruses, and other pathogens. IRAK1 is part of the IRAK family consisting of IRAK1, IRAK2, IRAK3, and IRAK4, and is activated by inflammatory molecules released by signaling pathways during pathogenic attack.

A number of IRAK1 inhibitors have been identified previously. Thus the IRAK1 inhibitor for use in the combination therapy of the present disclosure may be selected from the following compounds:

(1)

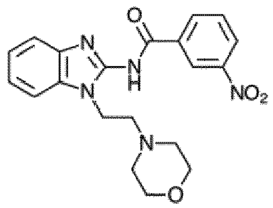

(2)

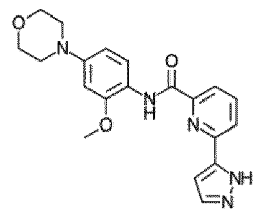

(3) Pacritinib

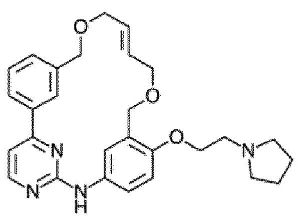

(4)

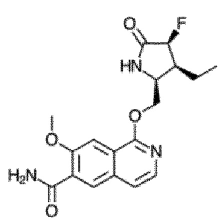

(5)

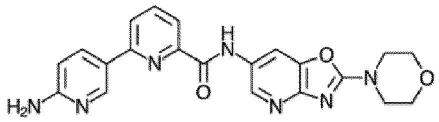

(6)

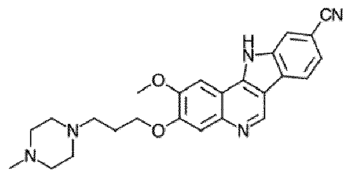

and pharmaceutically acceptable salts, solvates, hydrates, cocrystals, or prodrugs thereof. In one embodiment, the IRAK1 inhibitor is pacritinib.

HDAC Inhibitors

The therapeutic methods and combinations of the present invention also relate to a combination of an ADC which comprises an anti-CD22 antibody conjugated to a PBD dimer and (ii) an histone deacetylase inhibitor (HDI), such as a benzamide-based HDI, for example chidamide.

In one embodiment the HDI is primarily directed to (active against) class I HDACs, such as subtype 1, 2, 3 of class I HDACs.

In one embodiment, the HDI is selected from a benzamide and a hydroxamic acid.

In one embodiment, the HDI is selected from vorinostat, entinostat, panobinostat, romidepsin, belinostat, Mocetinostat, givinostat, pracinostat, chidamide, quisinostat and abexinostat.

Combination Therapy

The present disclosure relates to a combination therapy with both (i) an ADC which comprises an anti-CD22 antibody conjugated to a PBD dimer and (ii) (a) an IRAK1 inhibitor, such as pacritinib or (b) an HDAC inhibitor such as chidamide, are administered to a patient/subject in need thereof

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, regression of the condition, amelioration of the condition, and cure of the condition. Treatment as a prophylactic measure (i.e., prophylaxis, prevention) is also included.

The term "therapeutically-effective amount" or "effective amount" as used herein, pertains to that amount of an active compound, or a material, composition or dosage from comprising an active compound, which is effective for producing some desired therapeutic effect, commensurate with a reasonable benefit/risk ratio, when administered in accordance with a desired treatment regimen.

Similarly, the term "prophylactically-effective amount," as used herein, pertains to that amount of an active compound, or a material, composition or dosage from comprising an active compound, which is effective for producing some desired prophylactic effect, commensurate with a reasonable benefit/risk ratio, when administered in accordance with a desired treatment regimen.

Disclosed herein are methods of therapy. Also provided is a method of treatment, comprising administering to a subject in need of treatment a therapeutically-effective amount of an ADC and a therapeutically effective amount of an IRAK1 inhibitor such as pacritinib. The term "therapeutically effective amount" is an amount sufficient to show benefit to a subject. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors. The subject may have been tested to determine their eligibility to receive the treatment according to the methods disclosed herein. The method of treatment may comprise a step of determining whether a subject is eligible for treatment, using a method disclosed herein.

Compositions according to the present disclosure are preferably pharmaceutical compositions. Pharmaceutical compositions according to the present disclosure, and for use in accordance with the present disclosure, may comprise, in addition to the active ingredient, i.e. a conjugate compound, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which will typically be by injection, e.g. cutaneous, subcutaneous, or intravenous.

For intravenous, cutaneous or subcutaneous injection, or injection at the site of affliction, the active ingredient

will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

It will be appreciated by one of skill in the art that appropriate dosages of the ADC and other agent, and compositions comprising these active elements, can vary from subject to subject. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the subject. The amount of compound and route of administration will ultimately be at the discretion of the physician, veterinarian, or clinician, although generally the dosage will be selected to achieve local concentrations at the site of action which achieve the desired effect without causing substantial harmful or deleterious sideeffects.

Administration of the conjugate can be effected in one dose, continuously or intermittently (e.g., in divided doses at appropriate intervals) throughout the course of treatment. Methods of determining the most effective means and dosage of administration of conjugate are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

The ADC is typically administered intravenously whereas the inhibitors are typically administered orally, and typically they have different dosing intervals.

In one embodiment, the dose of ADC is in the range of from about 20 μg/kg to about 300 μg/kg, such as up to about 100 μg/kg or about 200 μg/kg and greater than about 30, 40 or 50 μg/kg, such as from about 50 μg/kg to about 200 μg/kg per treatment cycle. Typically, the dose per cycle is administered in one dose on one occasion during the treatment cycle. For example if the cycle is 7 days, the total dose may be given in one administration on day 1 and then again on day 8.

In some cases each treatment cycle is from 7 to 28 days, for example from 5 to 8 days or 12 to 16 days or 20 to 28 days, such as about one, two, three or four weeks.

Since initial clinical trials indicate rapid clearance of the antibody, in one embodiment, the treatment cycle is from 5 to 16 days such as once every 7 days or 14 days, such as about every 7 days. A more frequently administered dosage will generally be lower then a dose given less frequently e.g. where the treatment cycle is from 5 to 16 days, the dose per cycle may be from about 30 μg/kg to about 150 μg/kg, such as from about 40 μg/kg to about 120 μg/kg, about 40 μg/kg to about 100 μg/kg, or about 40 μg/kg to about 80 μg/kg.

The dose may also be expressed in terms of the weight, in μg, of the active substance (payload), i.e. the released PBD. The molecular weight of PBDs is typically in the order of 500 to 700 g/mol. SG3199 for example has a MW of 585 g/mol. Therefore since the doses given above are based on the total ADC, which due to the IgG and linker components has a MW of about 153 kDa (153,000 g/mol), the dose with respect to the PBD only (assuming 2 PBDs per IgG, i.e. a DAR of 2) will be less than 1/100 of the dose with respect to a full size IgG-based ADC.

Alternatively, the dose may be given as a flat dose (i.e. not based on weight). Based on a reference weight of 75 kg, the above weight-based doses can be converted to a flat dose. For example, about 20 μg/kg to about 300 μg/kg would be about 1.5 mg to about 22.5 mg.

In any of the above embodiments, the dose may be reduced after one or two cycles, for example reduced to about 25% to 75% of the starting dose or about 30% to 60%, such as to about 50%. In one embodiment this reduction occurs only once during the overall duration of treatment.

In one embodiment, the dose of IRAK1 inhibitor, such as pacritinib, is a total daily dose of from 100 to 600 mg. The dose may for example be given in two doses per day e.g. 100 or 200 mg twice daily.

In one embodiment, the dose of HDAC inhibitor, such as chidamide, is administered at a total daily dose from about 10 mg to about 40 mg. In certain embodiments, the HDI is administered at a total daily dose of about 10 mg. In certain embodiments, the HDI is administered at a total daily dose of about 15 mg. In certain embodiments, the HDI is administered at a total daily dose of about 20 mg. In certain embodiments, the HDI is administered at a total daily dose of about 25 mg (see WO2021/071809). The treatment frequency may be once or twice weekly, for example with an interval of at least 3 days.

## Treated disorders

The combination therapy described herein may be used to treat a proliferative disorder. The term "proliferative disorder" pertains to an unwanted or uncontrolled cellular proliferation of excessive or abnormal cells which is undesired,

such as, neoplastic or hyperplastic growth, whether *in vitro* or *in vivo.*

Examples of proliferative conditions include, but are not limited to, benign, pre-malignant, and malignant cellular proliferation, including but not limited to, neoplasms and tumours. The proliferative condition is typically characterized by expression of CD22. Accordingly, the proliferative conditions are generally haematological ones.

The target proliferative cells may be all or part of a solid tumour. The treated disorder may be, or be characterized by, an advanced solid tumour.

"Solid tumour" herein will be understood to include solid haematological cancers such as lymphomas (Hodgkin's lymphoma or non-Hodgkin's lymphoma).

Examples of disease or disorder to be treated herein include, but are not limited to non-Hodgkin's Lymphoma, including diffuse large B-cell lymphoma (DLBCL), follicular lymphoma, (FL), Burkitt's lymphoma (BL), Mantle Cell lymphoma (MCL), chronic lymphatic lymphoma (CLL), Waldenstroms Microglobulinemia, Burkitt's lymphoma, and Marginal Zone B-cell lymphoma (MZBL), and leukemias such as Hairy cell leukaemia (HCL), Hairy cell leukaemia variant (HCL-v), and Acute Lymphoblastic Leukaemia (ALL) such as Philadelphia chromosome-positive ALL (Ph+ALL) or Philadelphia chromosome-negative ALL (Ph-ALL).

The disease may be resistant, relapsed or refractory. As used herein, relapsed disease constitutes conditions in which a previously treated tumour which became undetectable by conventional imaging technology again becomes detectable; refractory disease a condition in which the cancer - despite anti-tumour therapy - continues to grow.

## Subject selection

In certain aspects, the subjects are selected as suitable for treatment with the methods described herein before the treatments are administered. In some aspects the methods described herein include the step of selecting suitable subjects. In some aspects the treatment methods described herein treat subjects that have been previously selected as suitable for treatment.

As used herein, subjects who are considered suitable for treatment are those subjects who are expected to benefit from, or respond to, the treatment. Individuals may have, or be suspected of having, or be at risk of having cancer. Individuals may have received a diagnosis of cancer. Typically the individual is an animal or human subject.

In some aspects, individuals are selected on the basis of the amount or pattern of expression of IRAK1 protein.

In some cases, individuals are selected on the basis they have, or are suspected of having, are at risk of having cancer, or have received a diagnosis of a proliferative disease characterised by the presence of a neoplasm comprising cells having a high level of expression of IRAK1 protein. The neoplasm may be composed of cells having a high level of expression of IRAK1 protein.

In some cases, individuals are selected on the basis they have, or are suspected of having, are at risk of having cancer, or have received a diagnosis of a proliferative disease characterised by the presence of a neoplasm comprising cells having a low level of expression of IRAK1 protein. The neoplasm may be composed of cells having a low level of expression of IRAK1 protein.

In some cases, expression of IRAK1 protein in a particular tissue of interest is determined. For example, in a sample of tumour tissue. In some cases, systemic expression of the target is determined. For example, in a sample of circulating fluid such as blood, plasma, serum or lymph.

In other aspects, the level of expression of IRAK1 protein is used to select a individual as suitable for treatment. Where the level of expression of IRAK1 protein is above a threshold level, the individual is determined to be suitable for treatment.

In some aspects, the amount of expression of IRAK1 protein must be above a threshold level to indicate that the individual is suitable for treatment.

In some aspects the sample is taken from a bodily fluid, more preferably one that circulates through the body. Accordingly, the sample may be a blood sample or lymph sample.

In some cases, the sample is a blood sample or blood-derived sample. The blood derived sample may be a selected fraction of an individual's blood, e.g. a selected cell-containing fraction or a plasma or serum fraction.

A selected cell-containing fraction may contain cell types of interest which may include white blood cells (WBC), particularly peripheral blood mononuclear cells (PBC) and/or granulocytes, and/or red blood cells (RBC). Accordingly, methods according to the present disclosure may involve detection of a first target polypeptide or nucleic acid in the blood, in white blood cells, peripheral blood mononuclear cells, granulocytes and/or red blood cells. In another aspect the sample is a biopsy of solid tissue.

The sample may be fresh or archival. For example, archival tissue may be from the first diagnosis of an individual, or a biopsy at a relapse. In certain aspects, the sample is a fresh biopsy.

The terms "subject", "patient" and "individual" are used interchangeably herein.

In some aspects disclosed herein, an individual has, or is suspected as having, or has been identified as being at risk of, a proliferative disease such as cancer. In some aspects disclosed herein, the individual has already received

a diagnosis of such a disease. A list of relevant diseases is provided herein.

In one embodiment the patient's disorder is resistant to monotherapy with an antibody drug conjugate (ADC) which comprises a pyrrolobenzodiazepine (PBD) dimer an anti-CD22 antibody.

Subjects may also be selected on the basis of CD22 expression as described above in this section for IRAK1.

Specific Embodiments

[1] A method of treating a proliferative disorder in a subject, said method comprising administering to the subject (i) an ADC which comprises an anti-CD22 antibody and a PBD dimer; and (ii)(a) an IRAK1 inhibitor, such as pacritinib or (b) a class I histone deacetylase inhibitor (HDI), such as chidamide (Tucinostat).

[2] The method according to [1] wherein the PDB dimer is of formula (I) or (III) as defined above.

[3] The method according to claim [1] or [2], wherein the linker is a cleavable linker, such as a linker comprising a cathepsin cleavable sequence.

[4] The method according to any of [1] to [3] wherein the the antibody comprises a VH domain having a CDR1 region with the sequence shown in SEQ ID NO.5; a CDR2 region with the sequence shown in SEQ ID NO.6; and a CDR3 region with the sequence shown in SEQ ID NO.7; and a VL domain having a CDR1 region with the sequence shown in SEQ ID NO.8; a CDR2 region with the sequence shown in SEQ ID NO.9; and a CDR3 region with the sequence shown in SEQ ID NO.10.

[5] The method of [4] wherein the antibody comprises a VH domain having the sequence according to SEQ ID NO. 1 and a VL domain having the sequence according to SEQ ID NO. 2.

[6] The method of any one of the preceding embodiments wherein the antibody comprises (i) heavy chains comprising an amino acid substitution of each of HC226 and HC229 according to the EU index as set forth in Kabat; (ii) light chains each having an amino acid substitution of the interchain cysteine residue κLC214 or λLC213 according to EU numbering; and (iii) heavy chains each retaining the unsubstituted interchain cysteine HC220 according to the EU numbering.

[7] The method of any one of the preceding embodiments where the ADC is ADCx22.

[8] The method of any one of the preceding embodiments where the IRAK1 inhibitor is selected from compounds (1) to (8) above, such as pacritinib, or a pharmaceutically acceptable salt, solvate, hydrate, cocrystal, or prodrug thereof.

[9] The method of any one of [1] to [7] where the HDI is selected from a benzamide, such as chidamide, or a hydroxamic acid, or a pharmaceutically acceptable salt, solvate, hydrate, cocrystal, or prodrug thereof.

[10] The method of any one of [1] to [7] where the HDI is selected from vorinostat, entinostat, panobinostat, romidepsin, belinostat, Mocetinostat, givinostat, pracinostat, chidamide, quisinostat and abexinostat.

[11] The method of any one of the preceding embodiments where the proliferative disorder is a tumour which comorises cells that express CD22.

[12] The method of [11] where the tumour comprises cells that express (typically overexpress) IRAK1.

[13] The method of [11] or [12] where the tumour is resistant to monotherapy with the ADC.

[14] The method of any one of the preceding embodiments wherein the proliferative disorder is selected from follicular lymphoma, marginal zone lymphoma, mantle cell lymphoma (MCL), diffuse large B-cell lymphoma (DLBCL), small lymphocytic lymphoma, hairy cell leukemia, and chronic lymphocytic leukemia.

[15] An ADC and an IRAK1 inhibitor as defined in any of any one of the preceding embodiments for use in a method of any one of the preceding embodiments.

[16] An ADC and an HDI as defined in any one of the preceding embodiments for use in a method of any one of the preceding embodiments.

[17] A kit comprising a first medicament comprising an anti-CD22 ADC; a package insert comprising instructions for administration of the first medicament according to a method of treatment as disclosed herein. The kit may further comprise a second medicament comprising (a) an IRAK1 inhibitor or (b) a class I Histone Deacetylase (HDAC) inhibitor (HDI), the package insert also comprising instructions for administration of the first medicament to an individual in combination with the second medicament for the treatment of a disorder.

**Sequence listing part of the description**

CD22

SEQ ID NO. 1 (Epratuzumab VH):

QVQLVQSGAE VKKPGSSVKV SCKASGYTFT SYWLHWVRQA PGQGLEWIGY INPRNDYTEY
NQNFKDKATI TADESTNTAY MELSSLRSED TAFYFCARRD ITTFYWGQG 109

SEQ ID NO. 2 (Epratuzumab VL):

DIQLTQSPSS LSASVGDRVT MSCKSSQSVL YSANHKNYLA WYQQKPGKAP KLLIYWASTR
ESGVPSRFSG SGSGTDFTFT ISSLQPEDIA TYYCHQYLSS WTFGQG 106

SEQ ID NO. 3 (EMabC220-HC):

QVQLVQSGAE VKKPGSSVKV SCKASGYTFT SYWLHWVRQA PGQGLEWIGY INPRNDYTEY 060
NQNFKDKATI TADESTNTAY MELSSLRSED TAFYFCARRD ITTFYWGQGT LVTVSSASTK 120
GPSVFPLAPS SKSTSGGTAA LGCLVKDYFP EPVTVSWNSG ALTSGVHTFP AVLQSSGLYS 180
LSSVVTVPSS SLGTQTYICN VNHKPSNTKV DKKVEPKS**C**D KTHT*V*PP*V*PA PELLGGPSVF 240
LFPPKPKDTL MISRTPEVTC VVVDVSHEDP EVKFNWYVDG VEVHNAKTKP REEQYNSTYR 300
VVSVLTVLHQ DWLNGKEYKC KVSNKALPAP IEKTISKAKG QPREPQVYTL PPSRDELTKN 360
QVSLTCLVKG FYPSDIAVEW ESNGQPENNY KTTPPVLDSD GSFFLYSKLT VDKSRWQQGN 420
VFSCSVMHEA LHNHYTQKSL SLSPG 445

SEQ ID NO. 4 (EMabC220-LC):

DIQLTQSPSS LSASVGDRVT MSCKSSQSVL YSANHKNYLA WYQQKPGKAP KLLIYWASTR
ESGVPSRFSG SGSGTDFTFT ISSLQPEDIA TYYCHQYLSS WTFGQGTKVE IKRTVAAPSV
FIFPPSDEQL KSGTASVVCL LNNFYPREAK VQWKVDNALQ SGNSQESVTE QDSKDSTYSL
SSTLTLSKAD YEKHKVYACE VTHQGLSSPV TKSFNRGE*S* 219

| | |
|---|---|
| SYWLH | SEQ ID NO.5 [VH CDR1] |
| YINPRNDYTE YNQNF KD | SEQ ID NO.6 [VH CDR2] |
| RDITTFY | SEQ ID NO.7 [VH CDR3] |
| KSSQSVLYSA NHKN | SEQ ID NO.8 [VL CDR1] |
| WASTRES | SEQ ID NO.9 [VL CDR2] |
| HQYLSSWT | SEQ ID NO.10 [VL CDR3] |

Some aspects and embodiments of the disclosure are described below in more detail with reference to the following examples, which are illustrative only and non-limiting. The Examples refer to the following figures:

Figure 1. Combination studies with ADCT-602. (A) Box plots of the combination of ADCT-602 with pacritinib in 8 lymphoma cell lines (4 high IRAK1 expression levels resistant to ADCT-602 [KARPAS-422, TOLEDO, SU-DHL-16, REC1] and 4 low IRAK1 expression levels sensitive to ADCT-602 [FARAGE, OCI-LY-1, VAL, and SP49]). Box plots represent the CI values obtained in individual cell lines. In each box plot, the line in the middle represents the median and the box extends from the 25th to the 75th percentile; the whiskers extend to the upper and lower adjacent values. Based on the Chou-Talalay CI, the effect of the combinations was defined as synergistic (CI < 0.9), additive (CI, 0.9-1.1), or antagonist (CI > 1.1). CI thresholds are visualized with dotted lines. (B) Cell surface expression of CD22 following treatment with chidamide analyzed by flow cytometry in Ramos, TMD8, and SU-DHL-4 cell lines. Data represent the mean ± standard error of at least 2 independent experiments, and they are expressed as MFI fold change (CD22 MFI chidamide/CD22 MFI DMSO). (C) In vitro cytotoxicity of ADCT-602 following pretreatment with chidamide for 7 days. Data are presented as the mean IC50 values of ADCT-602 from at least 3 independent experiments. Statistics were calculated using an unpaired t-test. CI, combination index; DMSO, dimethyl sulfoxide; IC50, 50% inhibitory concentration; IRAK1, interleukin 1 receptor-associated kinase 1; MFI, mean fluorescence intensity.

## EXAMPLES

Example 1 - Assessment of ADCT-602 efficacy in *in vivo* models

Ramos, WSU-DLCL2, and KARPAS-299 xenografts were established in 8- to 10-week-old female Fox Chase mice with severe combined immunodeficiency (Charles River Laboratories) by implanting $10^7$ cells subcutaneously (SC) into their flanks. When group mean tumor volumes reached approximately 116 to 132 $mm^3$, mice were randomly allocated into groups to receive the test agent or vehicle. Each animal was euthanized when its tumor reached the end point volume or at the study end. The time to end point (TTE) for analysis was calculated for each mouse by the following equation:

$$\text{TTE (days)} = ([\log_{10}(\text{end point volume} - \text{intercept*}]/\text{slope*})$$

where the * indicates the slope of the line obtained by linear regression of a log-transformed tumor growth data set.

The log-rank test was used to analyze the significance of differences in TTE between the 2 groups. For the disseminated xenografts model, $10^7$ REH cells were inoculated intravenously (IV) into 5- to 8-week-old female NOG mice (Taconic Biosciences). Test compounds were administered IV 4 days after tumor cell inoculation. Individual mice were euthanized, and an autopsy was performed when hind leg paralysis (a sign of leukemia development) occurred and/or the body weight decreased ≥20%. The study was terminated on day 62. The log-rank test was used to determine the significance of the differences in overall survival.

### DNA interstrand crosslink determination

Quantification of *in vitro* DNA interstrand crosslinks (ICLs) was performed using the single cell gel electrophoresis (Comet) assay.

*Results*

### Generation and characterization of ADCT-602

hLL2-C220, the antibody component of ADCT-602, was generated by introduction of mutations C214S in the light chain and C226V and C229V in the heavy chain hinge of the parental hLL2 antibody, which allowed for site-specific conjugation of the PBD dimer payload SG3249 to the cysteine at position 220 of each heavy chain. hLL2 is a clinically validated, humanized monoclonal antibody of the IgG1, kappa isotype specific for human CD22, which was derived from the murine antibody LL2.[16] Analysis of ADCT-602 by size exclusion chromatography showed that it was 98% monomeric (data not shown), while the DAR was determined to be ≈1.52 (data not shown). An in-depth analysis of ADCT-602 by LC-MS peptide mapping confirmed that conjugation only occurs on C220 of the heavy chain and that ADCT-602 lacked interchain disulfide bonds (data not shown).

### *In vitro* cytotoxicity of ADCT-602 in lymphoma and leukemia cell lines

ADCT-602 selectively inhibited the growth of a panel of 9 CD22-positive cancer cell lines derived from human lymphomas and leukemias (Daudi, Ramos, DO HH-2, GRANTA-519, NAMALWA, MEC-2, NALM-6, REH, and SEM), while no differential cytotoxicity with an isotype-control ADC (B12-C220-SG3249) was observed in 3 CD22-negative cell lines (KARPAS-299, SUP-T1, and KM-H2; Table 1). The PBD dimer cytotoxin SG3199 alone displayed potent cytotoxicity in all 12 cancer cell lines, independent of CD22 expression. No correlation was observed between CD22 expression and either ADCT-602 or SG3199 cytotoxicity, with Pearson correlation coefficient (r) of -0.47 (P = .12) and -0.037 (P= .91), respectively.

The *in vitro* cytotoxic activity of ADCT-602 was tested in a larger panel of lymphoma cell lines (n = 57), including both B- and T-cell-derived human lines (48 and 9 cell lines, respectively). ADCT-602 was more active in B-cell (median $IC_{50}$ 200 pM) than in T-cell (median $IC_{50}$ 1850 pM) lymphomas (Student t-test, P < .005) (Table 2), in line with CD22 expression patterns. The cytotoxic activity of ADCT-602 was similar in models derived from germinal center B-cell-like DLBCL, activated B-cell-like diffuse large B-cell lymphoma (ABC DLBCL), MCL, marginal zone lymphoma, chronic lymphocytic leukemia, and primary mediastinal B-cell lymphoma but was lower in Hodgkin lymphoma (Table 2). Among the B-cell lymphoma cell lines, the *in vitro* activity of ADCT-602 did not correlate with CD22 expression measured as cell surface protein levels (absolute quantitation, n = 45, $r$ = -0.238, and $P$ = .1), or RNA levels using either Illumina HT-12 microarrays (n = 42, $r$ = -0.28, and $P$ = .07) or the HTG EdgeSeq Oncology Biomarker Panel (n = 36, $r$ = -0.24, and

*P* = .16).

**The mode of action of ADCT-602**

CD22-expressing Ramos cells treated with ADCT-602 showed prominent cell surface binding (T 0 hours - data not shown). On incubation at 37°C, ADCT-602 showed cellular internalization and colocalization of ADCT-602 with LAMP-1 at both 1- and 2-hour time points (data not shown) with some intracellular ADC visible at 4 hours. There was no residual ADC staining at 24 hours, suggesting complete lysosomal degradation. In contrast, there was no evidence of membrane binding on the treatment of Ramos cells with an isotype-control ADC.

Following ADCT-602 binding, internalization, and trafficking to lysosomes, SG3249 was cleaved, releasing SG3199 inside the cells. DNA ICLs were measured using a modification of the single-cell gel electrophoresis (Comet) assay, and crosslinking was expressed as the percentage decrease in Olive tail moment (OTM) versus control irradiated cells. Following a 2-hour exposure of Ramos cells to ADCT-602, crosslink formation occurred. Immediately after the drug incubation period, a low level of ICLs (3%) was observed, which increased over time to near peak levels (≈50%) at 12 hours and persisted up to 48 hours. In contrast, SG3199 immediately induced a high level of ICLs (46%) after drug incubation, which further increased to 56% after 2 hours and persisted up to 48 hours. An equivalent concentration of the nonbinding control ADC produced DNA ICLs over 48 hours in the same pattern as ADCT-602 but at much lower levels (the peak was ≈18% at 12 hours). In the CD22-negative cell line (KARPAS-299), ADCT-602 produced a low level of DNA ICLs (≈15%) over 48 hours following a 2-hour treatment. In this cell line, SG3199 produced a high level of ICL (≈50%), similar to that observed in the Ramos cell line.

To determine whether ADCT-602 induces bystander killing of non-CD22 expressing cells, CD22-expressing Ramos cells were treated with ADCT-602 for 1, 2, or 3 days before transferring the conditioned media onto CD22-negative KARPAS-299 cells and incubating them for 96 hours. The ADCT-602-conditioned medium elicited a bystander effect after 1 day of pretreatment, as shown by a decrease in the percentage survival in the conditioned medium-treated KARPAS-299 cells, compared with the nonconditioned medium (97% to 78%, *P* = .001). The bystander effect increased after 2 to 3 days of preincubation in Ramos cells, as shown by a further decrease in the percentage of cell survival compared with the nonconditioned medium (66% and 62%, P < .0001). Conversely, when the conditioned medium from ADCT-602-treated, CD22-negative NCI-N87 cells was transferred to KARPAS-299, no significant change in the percentage of cell survival was measured compared with the nonconditioned medium.

**ADCT-602 has strong *in vivo* antitumor activity in CD22-positive lymphoma and leukemia xenograft models**

ADCT-602 demonstrated dose-dependent antitumor activity against both SC and disseminated tumor *in vivo* models. In the SC Ramos xenograft, a single dose of ADCT-602 at 0.3 and 1 mg/kg induced specific and dose-dependent antitumor activity compared with the vehicle and the isotype control ADC. The 1 mg/kg ADCT-602 dose resulted in 10/10 CRs and 9/10 tumor-free survivors at the end of the study (day 60).

In the SC WSU-DLCL2 xenograft model, a single dose of ADCT-602 at 1 and 3 mg/kg induced dose-dependent antitumor activity compared with the vehicle and the isotype control ADC. The 3 mg/kg ADCT-602 dose resulted in 3/10 partial responders, 7/10 CRs, and 2/10 tumor-free survivors at the end of the study (day 59).

In a disseminated B-ALL-derived xenograft model (REH), a single dose of ADCT-602 at 0.45 mg/kg and 1.3 mg/kg resulted in a dose-dependent extension of survival compared with the vehicle and the isotype control ADC. A single 1.3 mg/kg dose of ADCT-602 resulted in comparable survival time to the equivalent fractionated dose of ADCT-602 (0.45 mg/kg, every 4 days, for 3 times [Q4D×3]). Finally, in the SC KARPAS-299 xenograft model (a CD22-negative T-cell lymphoma-derived model), a single 1 mg/kg dose of ADCT-602 had no significant antitumor activity compared with the vehicle group at the end of the study (day 29).

In all models, treatments were well tolerated, with minimal body weight loss and no clinical observations reported.

<u>Example 2 - Gene expression signatures association analysis</u>

An exploratory analysis was conducted to define differentially expressed transcripts between groups of cell lines based on sensitivity to ADCT-602 using previously reported gene expression profiles obtained with the Illumina HT-12 microarray and with the HTG EdgeSeq Oncology Biomarker Panel (GSE94669), using *limma* (Ritchie et al. limma powers differential expression analyses for RNA-sequencing and microarray studies. Nucleic Acids Res. 2015;43(7):e47) with a cutoff of *P* < .05. Gene set enrichment analysis (GSEA) was performed using a ranked list of genes, which were based on the *limma*-defined log fold change and enriched gene sets were considered in the presence of a false discovery rate < 0.05.

*Results*

A gene expression signature association analysis was performed on the 48 B-cell lymphoma cell lines tested with ADCT-602 to search for pathways associated with resistance (median $IC_{50}$ value > 200 pM; n = 25) or sensitivity (median $IC_{50}$ value < 200 pM; n = 23) to ADCT-602. A total of 1207 genes were identified as down-regulated (logFC-) and 1104 genes as up-regulated (logFC+) in the resistant cell lines. To delineate the pathways associated with the different degrees of sensitivity to ADCT-602, a GSEA was performed on the preranked *limma* data. Transcripts up-regulated in the resistant cell lines were enriched for genes coding proteins involved in respiratory electron transport, oxidative phosphorylation, and proteasome. Conversely, transcripts up-regulated in cell lines sensitive to ADCT-602 were enriched for genes coding proteins involved in inflammation, p53 response, IL2/STAT5 signaling, hypoxia, and TGF-beta and interferon response. Interleukin-1 receptor-associated kinase 1 (IRAK1 - UniProt P51617) was among the differentially expressed genes associated with resistance to ADCT-602.

Example 3 - Combination Study with Pacritinib

Since IRAK1 was found to be among the differentially expressed genes associated with resistance to ADCT-602, we hypothesized that sensitivity to ADCT-602 could be restored by combining ADCT-602 with pacritinib (a selective Janus kinase 2, FMS-like tyrosine kinase 3, and IRAK1 multikinase inhibitor).

A combination of ADCT-602 and pacritinib was assessed in 8 lymphoma cell lines. Synergism assessment was performed by exposing cells (96 hours) to ADCT-602 (6.4 pM to 100 nM; 1- to 5-fold dilution) and pacritinib (27.4 nM to 20 $\mu$M; 1- to 3-fold dilution) alone or in combination, followed by MTT [3-(4,5-dimethylthiazol-2-yl)-2,5-diphenyltetrazolium bromide] assay. Chou-Talalay combination index (CI) values were determined. Combinations were defined as synergistic (median CI, < 0.9), additive (median CI, 0.9-1.1), or of no benefit/antagonist (median CI, > 1.1).

*Results*

In cells with low IRAK1 that were sensitive to ADCT-602, the combination was additive in 2/4 cell lines, while no benefit was observed in the other 2/4 cell lines (Figure 1A). In cell lines with high IRAK1 expression that were resistant to ADCT-602, the combination was synergistic in 2/4 cell lines, additive in 1/4, and had no beneficial effect in the remaining cell line. In MCL cells, the combination was not beneficial in cell lines with low IRAK1 that were sensitive to ADCT-602, while the combination reached synergism in cell lines with high IRAK1 expression that were resistant to ADCT-602.

Example 4 - Combination studies with chidamide

It has been suggested that chidamide, a histone deacetylase (HDAC) inhibitor, promotes the expression of CD22 on the surface of B-cell tumor cells and enhances the function of CD22 CAR-T. Therefore, we tested whether pretreatment of lymphoma cell lines with chidamide could enhance ADCT-602 activity by increasing CD22 expression.

Chidamide (Selleckchem) was dissolved in dimethyl sulfoxide (DMSO) according to the manufacturer's instructions. Cells were treated with chidamide (0.25 or 0.5 $\mu$M) for 7 days at 37°C in a 5% carbon dioxide-gassed, humidified incubator. On day 3, cells were centrifuged, and fresh medium containing the corresponding concentrations of chidamide was added. The control group was treated with equal volumes of DMSO.

CD22 cell surface expression after chidamide treatment was measured by flow cytometry. hLL2-C220 and B12 antibodies, followed by Alexa Fluor 488 anti-human secondary antibodies (Life Technologies, Thermo Fisher Scientific) or allophycocyanin anti-human secondary antibodies (BioLegend), were used for CD22 detection. Cells were analyzed using a CytoFLEX Flow Cytometer (Beckman Coulter), and data were analyzed with FlowJo software version 10.8.1 (BD Biosciences).

After treatment with chidamide or DMSO, cells were incubated with serial dilutions of ADCT-602 or the non-binding control ADC for 5 days. Cell viability was measured with the CellTiter 96 Aqueous One Solution Cell Proliferation Assay (Promega). Data were normalized to untreated control cells. The $IC_{50}$ values were determined using GraphPad software (GraphPad). The mean and standard errors of 3 independent $IC_{50}$ values were determined.

*Results*

Pretreatment with chidamide increased CD22 expression up to 2.4 times compared with DMSO-treated cells (Figure 1B), and ADCT-602 $IC_{50}$ improved up to 2.5-fold compared with cells pretreated with DMSO (Figure 1C). Conversely, no effect on the cytotoxicity of the isotype control ADC was seen across the 3 cell lines treated with chidamide (data not shown).

## Discussion

Here we report preclinical data for ADCT-602, a novel site-specific, PBD dimer-based, CD22-targeting ADC. ADCT-602 is based on reversed cysteine engineering of the IgG1 hinge, forcing conjugation on 1 cysteine of the heavy chain. The advantage of this format is that it provides a maximal DAR of 2, with no need for purification to remove any undesired higher-DAR species.

The cytotoxic payload tesirine, which uses SG3199, is also used in Lonca, which is approved by the FDA for use in R/R DLBCL based on strong single-agent activity in this hard-to-treat patient population, and in camidanlumab tesirine, which has encouraging phase 2 data in patients with R/R Hodgkin lymphoma. Previous attempts to develop CD19-based ADCs in R/R DLBCL employing other toxins had limited success, indicating that the PBD dimer toxin differentiates favorably in these patients. This favorable differentiation is also reflected in the safety profile. For example, inotuzumab ozogamicin and gemtuzumab ozogamicin, which use calicheamicin, have been linked to VOD, which is unlikely to occur with ADCT-602, based on the extensive clinical experience with Lonca and camidanlumab tesirine.

ADCT-602 showed potent *in vitro* activity in B-ALL and in a large panel of B-cell lymphoma subtypes, such as DLBCL and MCL but not in T-cell NHL, in line with the absence of CD22 on these cells. This indicates CD22 expression is required for the activity of ADCT-602, although no correlation was found between ADCT-602 cytotoxicity and CD22 copy number. This is similar to what was described for the CD22-specific, auristatin-based ADC, pinatuzumab vedotin (DCDT2980S). Evidence of rapid internalization of ADCT-602 was provided by the reduction in the intensity of membrane immunofluorescence staining on CD22-expressing Ramos cells, and LAMP-1 colocalization studies, suggesting that the processing of ADCT-602 is, at least in part, lysosomal. Similar to what was shown for Lonca, the time lag observed between the peak of DNA ICL formation by ADCT-602 and by SG3199 reflects the time taken for internalization and cellular processing of the ADC compared with the readily diffusible PBD dimer SG3199. The crosslinks were persistent and detected over the observation period of 48 hours. ADCT-602 and Lonca both exhibited bystander toxicity of targetnegative tumor cells. In medium transfer experiments, CD22-negative KARPAS-299 cells were killed by a soluble factor released from ADCT-602-treated Ramos cells into the medium. This soluble factor is assumed to be SG3199, released by lysosomal cleavage of ADCT-602 in the CD22-positive Ramos cells.

*In vivo,* ADCT-602 was remarkably effective against lymphoma and leukemia xenograft models in both the SC and disseminated settings. A single ADCT-602 dose selectively delivered sufficient cytotoxic agent, not only to delay tumor growth, but also to achieve sustained tumor regression and, in many cases, tumor eradication. Interestingly, in the disseminated REH model, a single 1.35 mg/kg ADCT-602 dose was equally active as a fractionated ADCT-602 dose (0.45 mg/kg; Q4D$\times$3). Overall, the *in vivo* activity of ADCT-602 was similar to that of Lonca.

An exploratory analysis comparing the expression profile of sensitive and resistant models identified IRAK1 as one of the differentially expressed genes associated with ADCT-602 resistance in lymphoma cell lines. The combination with pacritinib was evaluated in 8 lymphoma cell lines, 4 with high IRAK1 expression levels that were resistant to ADCT-602, and 4 with low IRAK1 expression levels that were sensitive to ADCT-602. The combination of ADCT-602 with pacritinib was more beneficial in cell lines with high IRAK1 expression that were resistant to ADCT-602 compared with cell lines with low IRAK1 expression that were sensitive to ADCT-602. These data suggest IRAK1 is a potential marker of ADCT-602 resistance. Additionally, treatment of B-cell lymphoma cell lines with the HDAC inhibitor chidamide produced an increased expression of membranous CD22, which resulted in increased but specific ADCT-602 cytotoxicity, indicating that HDAC inhibitors may be used as combination partners with anti-CD22 PBD-based ADCs described herein, such as ADCT-602.

Further, since ADCT-602 targets CD22 rather than CD19, it has the potential to be sequenced before or after other approved treatment modalities for B-cell malignancies that target CD19.

A number of publications are cited above to more fully describe and disclose the disclosures and the state of the art to which inventions herein may pertain. The entirety of each of the references mentioned in this disclosure are hereby is incorporated by reference.

Table 1. Mean CD22 molecules/cell in a panel of hematological tumor cell lines and mean $IC_{50}$ values of ADCT-602, an isotype-control ADC, and the PBD dimer cytotoxin SG3199.

| | Daudi | NAMALWA | Ramos | DO HH-2 | GRANTA-519 | MEC-2 | NALM-6 | REH | SEM | KM-H2 | SUP-T1 | KARPAS-299 |
|---|---|---|---|---|---|---|---|---|---|---|---|---|
| **Tumor type** | BL | BL | BL | FL | MCL | CLL | B-ALL | B-ALL | B-ALL | HL | T-LL | ALCL |
| **CD22 copy number ± SEM** | 122,938 ± 56,908 | 17,670 ± 221 | 43,005 ± 1117 | 202,470 ± 15,718 | 19,207 ± 282 | 69,291 ± 2670 | 14,825 ± 142 | 16,835 ± 99 | 24,704 ±335 | * | * | * |
| **SG3199 $IC_{50}$,** nM ± **SEM** | 0.008 ± 0.001 | 0.014 ± 0.001 | 0.016 ± 0.001 | 0.007 ± 0.001 | 0.002 ± 0 | 0.033 ± 0.009 | 0.002 ± 0 | 0.001 ± 0 | 0.005 ± 0.002 | 0.036 ± 0.005 | 0.026 ± 0.005 | 0.025 ± 0.001 |
| **ADCT-602 $IC_{50}$, nM ± SEM** | 0.07 ± 0.021 | 2.27 ± 0.052 | 0.06 ±0.021 | 0.06 ± 0.009 | 0.02 ± 0.0005 | 0.18 ±0.050 | 0.11 ± 0.018 | 0.05 ± 0.006 | 0.52 ± 0.080 | 11.4 ± 1.11 | 9.34 ± 1.02 | 51.7 ± 3.86 |
| **B12-C220-SG3249 $IC_{50}$, nM ± SEM** | 1.6 ± 0.145 | 2.63 ± 0.154 | 2.21 ± 0.797 | 0.66 ± 0.038 | 0.11 ±0.004 | 3.43 ± 0.665 | 0.50 ± 0.044 | 0.64 ± 0.054 | 1.65 t 0.148 | 6.29 ±0.184 | 6.98 ± 0.433 | 22.4 ± 2.43 |

Table 2. Median $IC_{50}$ values of ADCT-602 across B- and T-cell lymphoma and leukemia cell lines

| Histology | No. | Median $IC_{50}$ (pM) | 95% CI (pM) |
|---|---|---|---|
| B-cell lymphomas | 48 | 200 | 90-400 |
| MCL | 10 | 375 | 200-2000 |
| MZL | 6 | 62.5 | 21-155 |
| ABC-DLBCL | 6 | 395 | 55-1450 |
| GCB-DLBCL | 20 | 100 | 35-2500 |
| CLL | 2 | 230 | 135-325 |
| PMBCL | 1 | 35 | ND |
| HL | 3 | 4500 | 200-16,250 |
| T-cell lymphomas | 9 | 1850 | 700-15,000 |
| ALCL | 5 | 2850 | 325-15,000* |
| CTCL | 3 | 1750 | 790-45,000 |
| PTCL-NOS | 1 | 700 | ND |
| *Upper confidence limit held at the maximum of the sample. | | | |

ABC, activated B-cell; ALCL, anaplastic large cell lymphoma; CLL, chronic lymphocytic leukemia; CTCL, cutaneous T-cell lymphoma; DLBCL, diffuse large B-cell lymphoma; GCB, germinal center B-cell; HL, Hodgkin lymphoma; MCL, mantle cell lymphoma; MZL, marginal zone lymphoma; ND, not determined; PMBCL, primary mediastinal large B-cell lymphoma; PTCL-NOS, peripheral T-cell lymphoma-not otherwise specified.

## Claims

1. A method of treating a proliferative disorder in a subject which method comprises administering to the subject (i) an antibody drug conjugate (ADC), wherein the drug is a pyrrolobenzodiazepine (PBD) dimer and the antibody is an anti-CD22 antibody; and (ii) an IRAK1 inhibitor, which disorder is **characterized by** cells that express CD22.

2. A method according to claim 1 wherein the PBD dimer is of formula I:

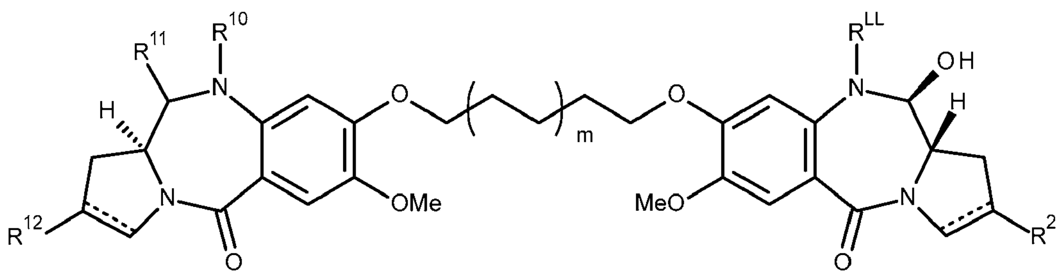

wherein:

(a) $R^{LL}$ is a linker for connection to Ab;
(b) (i) $R^{10}$ and $R^{11}$ together form a double bond between the nitrogen and carbon atoms to which they are attached; or (ii) $R^{10}$ is $R^{LLA}$ which is a linker for connection to Ab, and $R^{11}$ is OH, where $R^{LL}$ and $R^{LLA}$ may be the same or different; or (iii) $R^{10}$ is a capping group $R^C$ and $R^{11}$ is OH;
(c) m is 0 or 1; and
(d) when there is a double bond between C2 and C3, $R^2$ is methyl;

when there is a single bond between C2 and C3, $R^2$ is either H or

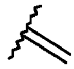

;

when there is a double bond between C2' and C3', $R^{12}$ is methyl;
when there is a single bond between C2' and C3', $R^{12}$ is H or

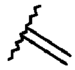

.

**3.** A method according to claim 1 wherein the PBD dimer is of formula (III):

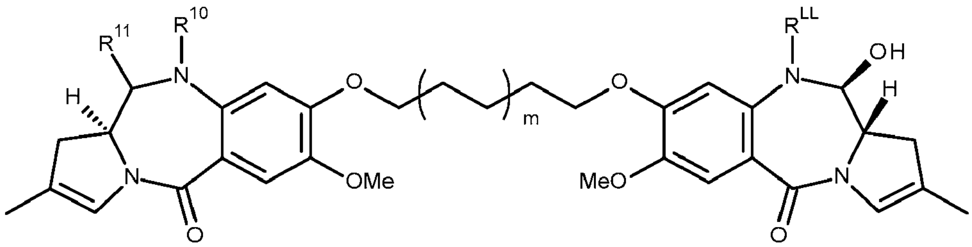

wherein:

(a) $R^{LL}$ is a linker for connection to Ab;
(b) (i) $R^{10}$ and $R^{11}$ together form a double bond between the nitrogen and carbon atoms to which they are attached; or (ii) $R^{10}$ is $R^{LLA}$ which is a linker for connection to Ab, and $R^{11}$ is OH; or (iii) $R^{10}$ is a capping group $R^C$, and $R^{11}$ is OH; and
(c) m is 0 or 1.

**4.** A method according to claim 2 or claim 3, wherein the linker is a cleavable linker, such as a linker comprising a cathepsin cleavable sequence.

**5.** A method according to any one of the preceding claims wherein the antibody comprises a VH domain having a CDR1 region with the sequence shown in SEQ ID NO.5; a CDR2 region with the sequence shown in SEQ ID NO.6; and a CDR3 region with the sequence shown in SEQ ID NO.7; and a VL domain having a CDR1 region with the sequence shown in SEQ ID NO.8; a CDR2 region with the sequence shown in SEQ ID NO.9; and a CDR3 region with the sequence shown in SEQ ID NO.10.

**6.** A method according to claim 5 wherein the antibody comprises a VH domain having the sequence according to SEQ ID NO. 1 and a VL domain having the sequence according to SEQ ID NO. 2.

**7.** A method according to any one of the preceding claims wherein the antibody comprises (i) heavy chains comprising an amino acid substitution of each of HC226 and HC229 according to the EU index as set forth in Kabat; (ii) light chains each having an amino acid substitution of the interchain cysteine residue κLC214 or λLC213 according to EU numbering; and (iii) heavy chains each retaining the unsubstituted interchain cysteine HC220 according to the EU numbering.

**8.** A method according to any one of the preceding claims where the ADC is ADCx22.

**9.** A method according to any one of the preceding claims where IRAK1 inhibitor is pacritinib or a pharmaceutically acceptable salt, solvate, hydrate, cocrystal, or prodrug thereof.

**10.** A method according to any one of the preceding claims where the proliferative disorder is a tumour which comprises cells that express CD22.

**11.** A method according to claim 10 where the tumour comprises cells that overexpress IRAK1.

**12.** A method according to any one of the preceding claims wherein the proliferative disorder is selected from follicular lymphoma, marginal zone lymphoma, mantle cell lymphoma (MCL), diffuse large B-cell lymphoma (DLBCL), small lymphocytic lymphoma, hairy cell leukemia, and chronic lymphocytic leukemia.

Figure 1

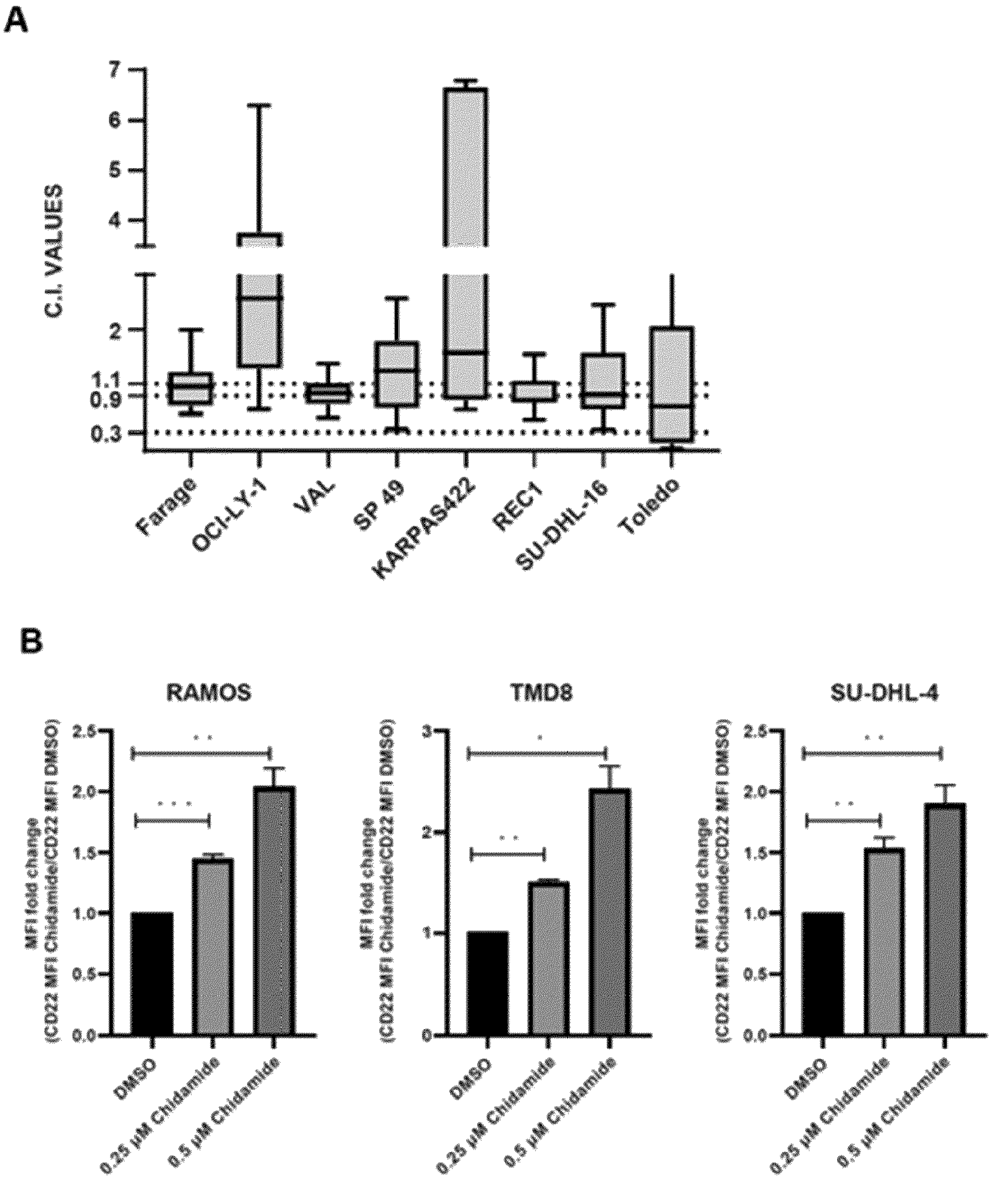
A
C.I. VALUES
7
6
5
4
2
1.1
0.9
0.3
Farage
OCI-LY-1
VAL
SP 49
KARPAS422
REC1
SU-DHL-16
Toledo
B
RAMOS
TMD8
SU-DHL-4
MFI fold change
(CD22 MFI Chidamide/CD22 MFI DMSO)
DMSO
0.25 µM Chidamide
0.5 µM Chidamide

**Figure 1 (cont.)**

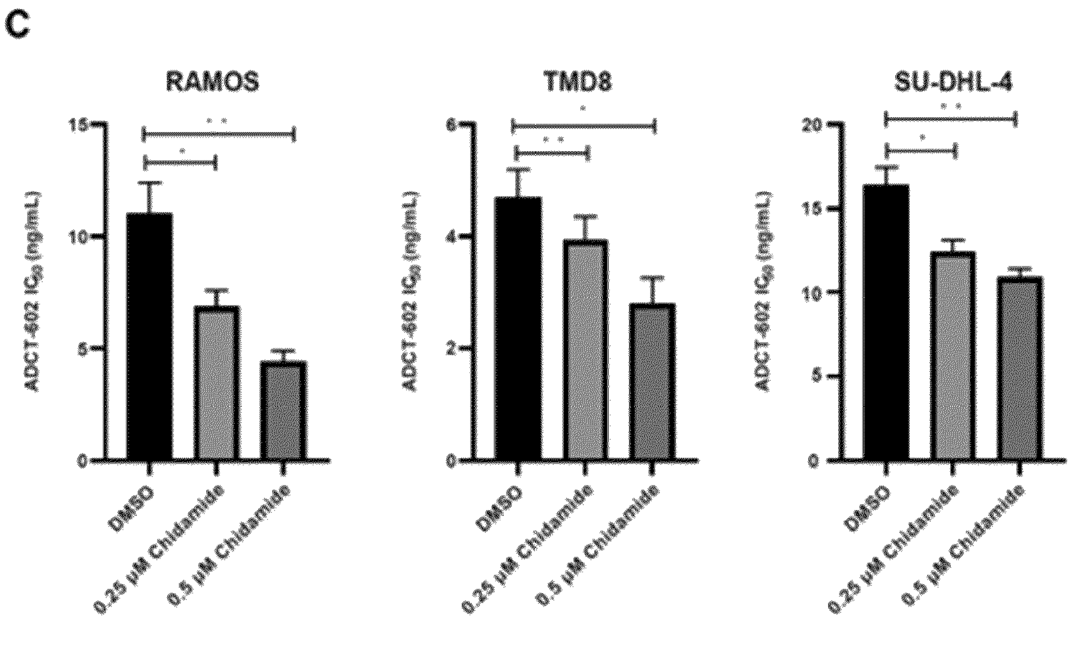

Europäisches Patentamt
European Patent Office
Office européen des brevets

# EUROPEAN SEARCH REPORT

Application Number
EP 23 15 4800

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br>A | WO 2018/193105 A1 (ADC THERAPEUTICS SA [CH]; MEDIMMUNE LTD [GB]) 25 October 2018 (2018-10-25)<br>* page 1, lines 9-10 *<br>* page 4, lines 14-24 *<br>* page 18, lines 26-28 *<br>* page 22, line 33 - page 23, line 11 *<br>* page 24, line 19 - page 25, line 8 *<br>* page 25, line 28 - page 58, line 35; examples 6, 9, 11 *<br>----- | 1-10,12<br>11 | INV.<br>A61K47/68<br>A61K31/4406<br>A61K45/06<br>A61P35/00 |
| Y<br>A | JACK W. SINGER ET AL: "Inhibition of interleukin-1 receptor-associated kinase 1 (IRAK1) as a therapeutic strategy", ONCOTARGET, vol. 9, no. 70, 7 September 2018 (2018-09-07), pages 33416-33439, XP055746632, DOI: 10.18632/oncotarget.26058<br>* section: "IRAK1 structure and inhibitors" *<br>* sections: "IRAK1 in oncology - solid tumors" and "IRAK1 in oncology - hematologic malignancies"; tables 2-3 *<br>----- | 1-10,12<br>11 | TECHNICAL FIELDS SEARCHED (IPC)<br>A61K<br>A61P |

The present search report has been drawn up for all claims

| Place of search | Date of completion of the search | Examiner |
|---|---|---|
| The Hague | 20 June 2023 | Fernandez Rodriguez |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 15 4800

This annex lists the patent family members relating to the patent documents cited in the above-mentioned European search report. The members are as contained in the European Patent Office EDP file on
The European Patent Office is in no way liable for these particulars which are merely given for the purpose of information.

20-06-2023

| Patent document cited in search report | Publication date | Patent family member(s) | Publication date |
|---|---|---|---|
| WO 2018193105 A1 | 25-10-2018 | AU 2018253951 A1 | 19-09-2019 |
| | | BR 112019021822 A2 | 26-05-2020 |
| | | CA 3057749 A1 | 25-10-2018 |
| | | CN 110869765 A | 06-03-2020 |
| | | EP 3612839 A1 | 26-02-2020 |
| | | JP 2020517640 A | 18-06-2020 |
| | | KR 20190137151 A | 10-12-2019 |
| | | US 2020405879 A1 | 31-12-2020 |
| | | US 2023149556 A1 | 18-05-2023 |
| | | WO 2018193105 A1 | 25-10-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 206166307 A
- US 7521541 B
- US 7723485 B
- WO 2009052249 A
- US 9889207 B
- WO 2018146188 A
- WO 2014057074 A
- WO 2018069490 A
- WO 2021071809 A

### Non-patent literature cited in the description


- **FRANZYK ; CHRISTIANSEN.** *Molecules,* 2021, vol. 26, 1292
- **DUBOWCHIK et al.** *Bioconjugate Chemistry,* 2002, vol. 13, 855-869
- **HAMBLETT.** *Clin. Cancer Res.,* 2004, vol. 10, 7063-7070
- **SANDERSON.** *Clin. Cancer Res.,* 2005, vol. 11, 843-852
- **FLYNN et al.** *Mol Cancer Ther,* 2016, vol. 15, 2709
- **VAN GEEL, R. et al.** *Bioconjugate Chemistry,* 2015, vol. 26, 2233-2242
- **TIBERGIEN et al.** *ACS Med. Chem. Lett.,* 2016, vol. 7, 983-987
- **RITCHIE et al.** limma powers differential expression analyses for RNA-sequencing and microarray studies. *Nucleic Acids Res.,* 2015, vol. 43 (7), e47